# EUROPEAN PATENT APPLICATION

(11) **EP 2 960 329 A1**
(43) Date of publication of application: **30.12.2015**
(21) Application number: 15151675.4
(22) Date of filing: 19.01.2015
(51) Int. Cl.: C12N 5/0775, C12N 5/00

(54) **Methods of upscaling mesenchymal stromal cell production, compositions and kit thereof**

(30) Priority: 24.06.2014 IN CH30722014
(71) Applicant: Stempeutics Research PVT. Ltd., Bangalore, 560066 Karnataka, (IN)
(72) Inventor: Kokundkar, Udaykumar, Whitefield, Bangalore 560 066, Karnataka (IN); Raj, Swathi Sundar, Whitefield, Bangalore 560 066, Karnataka (IN); Balasubramanian, Sudha, Whitefield, Bangalore 560 066, Karnataka (IN); Thej, Charan, Whitefield, Bangalore 560 066, Karnataka (IN); Mruthyunjaya, Ashwin Kunigal, Whitefield, Bangalore 560 066, Karnataka (IN); Damodaran, Devi, Whitefield, Bangalore 560 066, Karnataka (IN); Ramadasse, Balamurugan, Whitefield, Bangalore 560 066, Karnataka (IN); Bhagwat, Swaroop, Whitefield, Bangalore 560 066, Karnataka (IN); Majumdar, Anish Sen, Whitefield, Bangalore 560066 Karnataka (IN)
(74) Representative: V.O.

(57) **Abstract**

Present disclosure discloses a robust manufacturing process for consistent production of clinical grade Mesenchymal Stromal cells (MSCs). The process enables production of highly viable potent cells. The process steps relating to preparation of media, cell seeding, harvesting are fine tuned to achieve consistency in cell yield, superior cell viability, purity, improved cell proliferation, high cell recovery, low HLA-DR expression, reduction in culture duration. The viability and purity of cells are further improved by optimized wash process without cell loss/cell stress. The disclosure further provides a method of cyrostoring MSCs at high cell density without affecting the viability of cells. It further provides economical means to store and transport at -80°C.

## Description

### TECHNICAL FIELD

The present disclosure relates to a method of processing mesenchymal stromal cells to obtain viable and potent stem cell composition. In particular, the present disclosure relates to a process for obtaining consistency in high cell yield, increased viability, low HLA-DR and potency of mesenchymal stromal cells for clinical and therapeutic applications. Further the present disclosure also relates to an end to end production process and reduction in manipulation of the final composition before or during the administration for said applications.

### BACKGROUND

Human mesenchymal stromal cells (hMSCs) are present as a rare population of cells in bone marrow, representing 0.001 to 0.01% of the nucleated cells, but they can rapidly grow and expand in culture without losing their stemness. The hMSCs, with their attributes of (1) ease of isolation, (2) high expansion potential, (3) genetic stability, (4) reproducible characteristics and (5) compatibility with tissue engineering principles, have the potential to enhance repair in many damaged tissues.

MSCs and MSC-like cells have now been isolated from various tissues other than the bone marrow which includes adipose tissue, amniotic fluid, periostium and fetal tissues, and show phenotypic heterogeneity. Mesenchymal stromal cells obtained from human sources such as bone marrow (hBMSCs) have been widely studied because of their relative easy access and differentiation potential to the osteogenic, adipogenic and chondrogenic lineages, and other kind of tissues or cells, including hepatocytes, cardiomyocytes and neurons. Their multipotentiality and self-renewal has increased the attention to this stem cell as a self-renewing cell source with applications in regenerative medicine. In addition, their isolation based on the adherence to the culture substrates constitutes a straightforward strategy for elimination of non-mensenchymal lineages, reducing the dependency on complex cell isolation methods which rely on the expression of specific surface markers.

Further, adherent mesenchymal stem cells for therapeutic use are typically produced using planar technologies (flasks). Ten-layer stacks or vessels have been used to progress several allogeneic cell therapy products into early, mid to late-stage clinical development. Scaling up traditional flask-based culture processes from laboratory scale usually involves commercially available stacked-plate systems such as Corning Cell STACKS. These multilayer vessels have been used for large-scale cell culture. Traditional 10-layer Cell STACKS have been adopted for manufacturing processing and are being used as a platform in the good manufacturing practice (GMP) production of allogeneic mesenchymal stromal cells for therapeutic applications.

Although, cell therapies that employ mesenchymal stromal/stem cells show great promise, the limitation with bone marrow derived MSCs is their small quantity, but whereas for clinical application large quantity of MSCs are required. As research in Mesenchymal Stromal cells (MSCs) translates into commercial products, another major obstacle in bringing cell-based products into the market is the need for robust and reproducible production process and quality of cryopreservation media and storage containers for biological systems.

MSCs are multipotent and are capable of differentiating into osteoblasts, chondrocytes, and adipocytes. BM-derived MSCs have been more widely used and there is a much greater quantity of data regarding their clinical safety and practice. To meet this clinical demand, a fast and robust MSC expansion method is required for manufacturing and storage of a large-scale inventory of a uniform, off-the-shelf product. In the prior art, PCT Application no. PCT/US2009/053891 discloses pharmaceutical compositions comprising mesenchymal stem cells that have reduced immunogenicity and methods of manufacturing the same using centrifugal filtration. This patent application is on method of manufacturing one or more purified MSC pharmaceutical composition by utilizing centrifugal filtration but do not disclose critical quality parameters of cell based products such as cell yield and HLA-DR. Further purified MSC composition comprises of less than 55ug/ml residual BSA. However, this does not provide insights to achieve increase in cell yield, viability and quality parameters like low HLA-DR and purity. US Patent Application no. 13/267, 363 provides preparations of mesenchymal stem cells with important therapeutic potential, with a structural and functional phenotype that is stable in culture, methods of expanding MSCs to produce clinical scale therapeutic preparations and medical uses thereof. Another PCT Application no. PCT/US2011/022054 focuses on methods and apparatus for manufacturing cell therapy product and washing the cell therapy product by volume reduction using TFF the technology to reduce the BSA content in a subsequent procedure. However no data on cell loss during TFF procedure is provided and moreover, BSA levels is in the range of about 100ng/ml which is considered not suitable for therapeutic applications. Thus, there exists a need for a process that would ensure uniform distribution of growth factors (bFGF) to media for uniform cell distribution and proliferation, a process for improving cell yield, viability, consistency, with reduced culture duration and cell loss, being devoid of impurities and a method of preserving the cell product to improve its shelf life.

Major hindrance seen in the conventionally followed methods of stem cell expansion and final delivery of the cell- based product are:
- Lack of consistent production to obtain large cell yield in short duration;
- High cell loss during wash and centrifugation process leading to low cell yield and viability;
- Costly storage and transportation;
- Requirement of certain amount of reconstitution of the cryopreserved cells before administration to the patients; and
- Further manipulation of cryopreserved cells at the hospital thus making the product vulnerable to loss of cells or loss of sterility.

The present disclosure thus provides a process and means for overcoming the above problems. Thus, the present disclosure makes an attempt to overcome the increasing need for the development of comprehensive production process and storage of final product. The focus is therefore on process improvement and strategy for developing ready to use allogeneic mesenchymal stromal/stem cells for cell therapies, which are the most widely used cell types in cell therapy today.

### STATEMENT OF THE DISCLOSURE

The present disclosure relates to a process for culturing of mesenchymal stromal cells, said process comprising act of: allowing the mesenchymal stromal cells cultured till a first predetermined passage, to expand to a second predetermined passage in presence of culture media comprising basic fibroblast growth factor (bFGF), wherein said expansion is carried out by contacting the cells with said culture media when said cells achieve at least one pre-determined confluency; and wherein the process increases number of the cells at the end of the second predetermined passage by 2000 folds when compared to number of the cells at the first predetermined passage.

The present disclosure also relates to a method of reducing amount of BSA to a level of below 50ng/ml for a predetermined dosage formulated for clinical or therapeutic application, said dosage comprising mesenchymal stromal cells obtained by culturing of said cells, said method comprising act of subjecting the composition to a combination of centrifugation and washing with phosphate buffer saline to reduce the amount of said BSA.

The present disclosure also relates to a process for storing mesenchymal stromal cells, said process comprising step of subjecting the cells to a freezing mixture at a cell density ranging from about 5 million cells to about 25 million cells per ml of the freezing mixture, and storing the cells at a temperature ranging from about -75 degrees Celsius to about -85 degrees Celsius or at a temperature ranging from about -190 degrees Celsius to about -200 degrees Celsius.

### BRIEF DESCIPTION OF ACCOMPANYING FIGURES

In order that the disclosure may be readily understood and put into practical effect, reference will now be made to exemplary embodiments as illustrated with reference to the accompanying figures. The figures together with a detailed description below, are incorporated in and form part of the specification, and serve to further illustrate the embodiments and explain various principles and advantages, in accordance with the present disclosure where:
**Figure 1A:** shows mesenchymal stromal cell yield per batch at passage 4(P4).
**Figure 1B:** shows the mean average cell yield from the 5 individual batches of Figure 1A.
**Figure 2****:** shows total cell yield at P5 from different batches.
**Figure 3A****:** shows graphical representation of percentage viability by 7AAD of P5 harvest from different batches of the current disclosure.
**Figure 3B****:** shows graphical representation of percentage viability by 7AAD of post thaw IMP of the current disclosure showing prominent consistency in the post thaw viability.
**Figure 3C****:** shows mean average of percentage viability by 7AAD of post thaw IMP of the 5 individual production batches of Figure 3B.
**Figure 4A****:** shows representation of post-harvest HLA-DR expression in different production batches of the current process which shows a significant lower expression of HLA-DR.
**Figure 4B****:** shows mean average of post-harvest HLA-DR expression of the 5 individual production batches of Figure 4A.
**Figure 4C****:** shows representation of post-thaw HLA-DR expression in different production batches using the process of the instant disclosure.
**Figure 4D****:** shows mean average of post-thaw HLA-DR expression of 5 individual production batches of Figure 4C.
**Figure 5A****:** shows preparation of bFGF master mix.
**Figure 5B****:** shows preparation of seed master mix.
**Figure 6A****:** shows consistency in cell yield in different production batches before ('Previously Known') and after ('New Process' of the instant disclosure) implementing bFGF and seed master mix.
**Figure 6B****:** shows consistency in HLA-DR expression in different production batches before ('Previously Known') and after ('New Process' of the instant disclosure) implementing bFGF and seed master mix
**Figure 7A:** shows the impact of feeding schedule based on confluency on cell yield of individual 10 Cell STACKs of different batches of the current process based on confluencies (2), compared with a previously known process wherein culturing is carried out based on no. of days (1).
**Figure 7B:** shows the HLA-DR expression in individual 10 Cell STACKs of different production batches of the current process based on confluencies (2), compared with a previously known process wherein culturing is carried out based on no. of days (1).
**Figure 8A****:** shows the comparison of BSA level in the IMP by following the washing steps indicated in the present disclosure (i.e. wash I with 200 ml DPBS and wash II with 90 ml DPBS) with respect to the BSA levels in the IMP obtained by following previously known washing steps (i.e. wash I and wash II with 200 ml of DPBS each).
**Figure 8B****:** shows the comparison of cell loss in the final product of the present disclosure as obtained by following the washing steps indicated in the present disclosure (i.e. wash I with 200 ml DPBS and wash II with 90 ml DPBS) with respect to the cell yield of the final product obtained by following previously known washing steps (i.e. wash I and wash II with 200 ml of DPBS each).
**Figure 8C****:** shows the impact of washing and centrifugation on post thaw cell recovery of final product from the current process, when compared to previously known wash process.
**Figure 9A****:** shows the post-thaw viability by 7AAD of BM-MSCs following cryopreservation with CS5 Cell viability was assessed at a cryopreservation age (time point) of one week, one month, two months, three months and six month, with five different freezing concentration of 5, 10, 12.5, 15 and 25 million cells per ml of the CS5.
**Figure 9B****:** shows post-thaw Total cell recovery (TCR) of BM-MSCs following cryopreservation with CS5. TCR was assessed by staining the cells with trypan blue at a cryopreservation age (time point) of one week, one month, two months, three months and six months. The sum of total viable and
non-viable was counted as TCR. TCR of Five different freezing concentration of 5, 10, 12.5, 15 and 25 million cells per ml of the CS5 were observed.
**Figure 10A****:** shows the viability by 7AAD of 25M cells per ml of CS5 freezing mixture of different dose of 25M dose, 50M dose and 75M dose for 6 months stored at -196°C.
**Figure 10B**: shows the cell recovery of 25M cells per ml of CS5 freezing mixture of different dose of 25M dose, 50M dose and 75M dose for 6 months stored at -196°C.
**Figure 10C****:** shows the viability by 7AAD for 25M cells per ml of CS5 freezing mixture of different dose of 100 and 200 million for 12 months stored at -196°C. The Y-axis represents % of 7AAD viability.
**Figure 11A****:** shows the immunosuppressive property of IMP by the ability to suppress a mixed lymphocyte reaction (MLR). Data is an average of six individual batches.
**Figure 11B****:** shows the estimation of PGE-2 in supernatants from IMP-MLR co-cultures. Data is an average of five individual batches.
**Figure 11C****:** shows the correlation between the amount of PGE-2 produced and the immunosuppressive capacity of the IMP.
**Figure 12****:** shows the angiogenic potency of the IMP in terms of production of VEGF when compared to that of a cell-based product produced by a previously known process.
**Figure 13****:** shows the chondrocyte potency of the IMP in terms of production of sGAG when compared to that of a cell-based product produced by a previously known process
**Figure 14A****:** shows the post thaw viability by 7AAD of 3rd month time point of direct stability and accelerated stability study.
**Figure 14B****:** shows post thaw proliferation of -80°C direct (right side) and accelerated stability (left side) study of 3 months.
**Figure 14C****:** shows post thaw CFU-F of -80°C direct and accelarated (control) stability study.
**Figure 14D****:** shows trilineage differentiation of -80°C direct and accelarated stability study.

### DETAILED DESCRIPTION OF THE DISCLOSURE

The present disclosure relates to a process for culturing of mesenchymal stromal cells, said process comprising act of:
a. allowing the mesenchymal stromal cells cultured till a first predetermined passage, to expand to a second predetermined passage in presence of culture media comprising basic fibroblast growth factor (bFGF);
wherein said expansion is carried out by contacting the cells with said culture media when said cells achieve at least one pre-determined confluency; and wherein the process increases number of the cells at the end of the second predetermined passage by 2000 folds when compared to number of the cells at the first predetermined passage.

In an embodiment of the present disclosure, the increase in the number of cells occurs in a maximum period of 21 days.

In another embodiment of the present disclosure, the cells at the first predetermined passage are allowed to expand to the second predetermined passage by consecutive passages; and wherein the first predetermined passage ranges from passage 2 to passage 9; and the second predetermined passage ranges from passage 5 to passage 10.

In another embodiment of the present disclosure, the first predetermined passage is passage 3; and the second predetermined passage is passage 5.

In another embodiment of the present disclosure, number of the cells seeded for said expansion ranges from about 0.6 million to about 0.7 million cells; and number of the cells obtained after said expansion is at least about 1800 million cells; and wherein the process increases the number of cells by at least 2000 folds.

In another embodiment of the present disclosure, components of the culture media are Dulbecco's Modified Eagle Medium-KnockOut [DMEM-KO] at a concentration ranging from about 75% to 95%, Fetal Bovine Serum [FBS] at a concentration ranging from about 5% to 15%, Glutamine at a concentration ranging from about 0.5% to 2%, Pen-Strep having penicillin at a concentration of about 50 to about 100 U/ml and streptomycin at a concentration of about 50 to about 100 µg/ml, and basic Fibroblast Growth Factor (bFGF) at a concentration ranging from about 0.5 ng/ml to 5 ng/ml.

In another embodiment of the present disclosure, the culture media is prepared by a process of master mixing of the components in a manner so as to avoid volumetric error and provide uniform distribution of the bFGF within multiple aliquots of the media, said process comprising acts of:
a. preparing 'X' Ltrs of the culture media comprising the DMEM-KO, FBS, Glutamine and Pen-Strep, devoid of bFGF, and dispensing into 'X' 1Ltr containers and labelling the containers from 1 to 'X';
b. withdrawing 'Z' ml of media from container No. 1, and dispensing into a new sterile container labelled No. 'X+1';
c. adding a predetermined quantity of bFGF to the 'X minus(-) Z' ml of media of container No. 1 and mixing well to obtain bFGF master mix;
d. separately withdrawing an equal quantity of media from each of the containers labelled 2 to 'X' and adding to the 'Z' ml of media in container No. 'X+1', thereby making the total media volume of container No. 'X+1' as 'B' ml; and
e. separately withdrawing a second predetermined quantity of bFGF master mix obtained in step (c) and adding to each 'B' ml media in containers 2 to 'X' thereby making the volume in each of the said containers equal and preparing multiple aliquots of the culture media.

In another embodiment of the present disclosure, the at least one pre-determined confluency is selected from ranges of confluencies comprising about 30% to about 40%; about 40% to about 50%; and about 60% to about 70%, or any combination thereof.

In another embodiment of the present disclosure, the expansion by contacting the cells with the culture media comprises acts of:
a. expanding the cultured cells into a consecutive passage by seeding the cells at a seeding density ranging from about 1000 cell/cm² to about 7000 cells/cm² and providing the culture media to the seeded cells; and
b. allowing the cells to reach a predetermined confluency of about 40% to about 50%, and replacing the culture media to obtain the expanded cells;
wherein, the culture media employed throughout the expansion is at a concentration ranging from about 0.15 to about 0.35 ml/ cm².

In another embodiment of the present disclosure, the expansion increases the number of cells by at least 30 folds.

In another embodiment of the present disclosure, the expansion by contacting the cells with the culture media comprises acts of:
a. expanding the cultured cells into a consecutive passage by seeding the cells at a seeding density ranging from about 1000 cell/cm² to about 7000 cells/cm² and providing the culture media to the seeded cells;
b. allowing the cells to reach a predetermined confluency of about 30% to about 40%, and adding the culture media to the cells without removing the spent media; and
c. allowing the cells to reach a second predetermined confluency of about 60% to about 70%, and replacing the culture media to obtain the expanded cells;
wherein, the culture media employed throughout the expansion is at a concentration ranging from about 0.15 to about 0.35 ml/ cm².

In another embodiment of the present disclosure, the expansion increases the number of cells by at least 40 folds.

In another embodiment of the present disclosure, the expansion by contacting the cells with the culture media comprises acts of:
a. expanding the cultured cells into a consecutive passage by seeding the cells at a seeding density ranging from about 1000 cell/cm² to about 7000 cells/cm² and providing the culture media to the seeded cells;
b. allowing the cells to reach a predetermined confluency of about 40% to about 50%, and replacing the culture media of the cells;
c. further expanding the cells of step (b) into a second consecutive passage by seeding the cells at a seeding density ranging from about ranging from about 1000 cell/cm² to about 7000 cells/cm² and providing the culture media to the seeded cells;
d. allowing the cells to reach a predetermined confluency of about 30% to about 40%, and adding the culture media to the cells without removing the spent media; and
e. allowing the cells to reach a second predetermined confluency of about 60% to about 70%, and replacing the culture media to obtain the expanded cells;
wherein, the culture media employed throughout the expansion is at a concentration ranging from about 0.15 to about 0.35 ml/ cm².

In another embodiment of the present disclosure, the expansion increases the number of cells by at least 2000 folds.

In another embodiment of the present disclosure, the cells obtained after said culturing are subjected to freezing with a freezing mixture at cell density ranging from about 5 million cells to about 25 million cells per ml of the freezing mixture.

In another embodiment of the present disclosure, the freezing is carried out at a temperature ranging from about -75 degrees Celsius to about -85 degrees Celsius or at a temperature ranging from about - 190 degrees Celsius to about -200 degrees Celsius; and wherein the freezing mixture comprises a cryopreservant at a concentration ranging from about 2% to about 15%, preferably about 5%; and wherein the cryopreservant is preferably DMSO.

In another embodiment of the present disclosure, the freezing at said cell density allows retention of viability and post-thaw functionality.

In another embodiment of the present disclosure, said process comprises act of:
a. allowing the mesenchymal stromal cells cultured till passage 3, to expand to passage 5 in presence of culture media comprising basic fibroblast growth factor (bFGF);
wherein said expansion is carried out by contacting the cells with said culture media when said cells achieve at least one pre-determined confluency; and wherein the process increases number of the cells at the end of passage 5 by at least 2000 folds when compared to number of the cells at the passage 3.

In another embodiment of the present disclosure, the expansion by contacting the cells with the culture media comprises acts of:
a. expanding the cells cultured to passage 3 to expand into passage 4 by seeding the passage 3 cells at a seeding density ranging from about 1000 cell/cm² to about 7000 cells/cm² and providing the culture media to the seeded cells;
b. allowing the cells to reach a predetermined confluency of about 40% to about 50%, and replacing the culture media;
c. further expanding the cells obtained at the end of step (b) into passage 5 by seeding the cells at a seeding density ranging from about 1000 cell/cm² to about 7000 cells/cm² and providing the culture media to the seeded cells;
d. allowing the cells to reach a predetermined confluency of about 30% to about 40%, and adding the culture media without removing the spent media; and
e. allowing the cells to reach a second predetermined confluency of about 60% to about 70%, and replacing the culture media to obtain the expanded cells;
wherein, the culture media employed throughout the expansion is at a concentration ranging from about 0.15 to about 0.35 ml/ cm².

In another embodiment of the present disclosure, the cells obtained upon said culturing process are further subjected to a combination of centrifugation and wash process prior to being formulated into a predetermined dosage form for clinical or therapeutic application; and wherein the said combination reduces the levels of bovine serum albumin [BSA] present to an amount of below 50ng/ml for said predetermined dosage, wherein said BSA is provided to the cells during the culturing process by the FBS in the culture media.

The present disclosure also relates to a method of reducing amount of BSA to a level of below 50ng/ml for a predetermined dosage formulated for clinical or therapeutic application, said dosage comprising mesenchymal stromal cells obtained by culturing of said cells, said method comprising act of subjecting the composition to a combination of centrifugation and washing with phosphate buffer saline to reduce the amount of said BSA.

In another embodiment of the present disclosure, the BSA at an amount of above 50ng/ml is provided to the cells during the culturing of said cells involving use of components of bovine origin; and wherein the level of below 50ng/ml is regardless of the number of the cells in the dosage.

In another embodiment of the present disclosure, the method comprises acts of:
a. subjecting the cells to a first centrifugation at speed ranging from about 1200 rpm to about 1800 rpm for time period ranging from about 7 minutes to about 10 minutes;
b. subjecting the centrifuged cells to a first washing with about 20 ml DPBS for cell numbers in the range of about 0.6million to about 6 million, and re-centrifuging the washed cells at speed ranging from about 1200 rpm to about 1800 rpm for time period of about 10 minutes;
c. subjecting the re-centrifuged cells to a second washing with about 7 to about 9 ml of DPBS for cell numbers in the range of about 600 to about 6 million, followed by optional pooling of the cell samples; and
d. re-centrifuging the washed cells from step (c) at speed ranging from about 1200 rpm to about 1800 rpm for time period ranging from about 5 minutes to about 7 minutes to obtain the cells having BSA at a level of below 50ng/ml.

The present disclosure further relates to a process for storing mesenchymal stromal cells, said process comprising step of subjecting the cells to a freezing mixture at a cell density ranging from about 5 million cells to about 25 million cells per ml of the freezing mixture, and storing the cells at a temperature ranging from about -75 degrees Celsius to about -85 degrees Celsius or at a temperature ranging from about -190 degrees Celsius to about -200 degrees Celsius.

In another embodiment of the present disclosure, the storing of mesenchymal stromal cells in a freezing mixture at a cell density ranging from about 5 million cells to about 25 million cells per ml of the freezing mixture, and at a temperature ranging from about -75 degrees Celsius to about -85 degrees Celsius, allows retention of viability and post-thaw functionality for a period of at least 1 week without liquid nitrogen.

In another embodiment of the present disclosure, the storing of mesenchymal stromal cells in a freezing mixture at a cell density ranging from about 5 million cells to about 25 million cells per ml of the freezing mixture, and at a temperature ranging from about -190 degrees Celsius to about -200 degrees Celsius, allows retention of viability and post-thaw functionality for a period of at least 1 month.

In another embodiment of the present disclosure, the freezing mixture comprises a cryopreservant at a concentration ranging from about 4% to about 11%, preferably about 5%; and wherein the cryopreservant is preferably DMSO.

In another embodiment of the present disclosure, the freezing at said cell density allows retention of viability and post-thaw functionality.

In another embodiment of the present disclosure, the mesenchymal stromal cells are obtained by culturing of said cells by process as described above.

In another embodiment of the present disclosure, the process eliminates reconstitution of the cells prior to administration for a therapeutic purpose to a subject in need thereof.

The present disclosure relates to a large scale manufacturing process of mesenchymal stromal cells (MSCs) having reduced culture duration, for producing consistent, viable and high cell yield.

An embodiment of the present disclosure relates to a process for production of MSCs with low HLA-DR expression.

Another embodiment of the present disclosure provides an enhanced wash process to remove impurities and reduce/minimize the cell loss during centrifugation.

Yet another embodiment of the present disclosure relates to a process for production of immunosuppressive and immunopotent MSC population for preparing final cell composition which is highly effective for clinical applications.

The present disclosure further provides a method of cryopreserving MSCs at high cell density in low volume without affecting the post thaw viability.

In another embodiment of the present disclosure, a method is disclosed to cryopreserve MSCs at high density in a cryopreservation solution without affecting the post-thaw cell viability and potency.

An embodiment of the present disclosure discloses a means for storage, transportation and delivery of final cell product without the need of reconstitution or manipulation.

In a non-limiting embodiment of the present disclosure, the term mesenchymal stromal cells (MSCs) as used in this disclosure includes but is not limited to mesenchymal stem cells and adult stem cells derived from various sources including but not limiting to bone marrow, adipose tissue, Wharton's Jelly, Dental pulp etc. In an embodiment of the present disclosure, the MSCs within the purview of the present disclosure include mesenchymal stem cells derived preferably from bone marrow.

In an embodiment, the mesenchymal stromal cells employed in the present disclosure are isolated from different human sources such as bone marrow, adipose tissue, Wharton's Jelly, Dental pulp, etc by established conventional methods in the art. In an embodiment of the present disclosure, for employing bone marrow derived MSCs, the bone marrow obtained from healthy volunteers are diluted in diluting culture medium comprising DMEM-KO, FBS, glutamine and penicillin-streptomycin instead of using saline/Hank's solutions. The complete culture medium is used from the step of isolation and throughout the process of culturing, harvesting and cryopreservation to reduce any cell stress.

Still another embodiment of the present disclosure relates to an end-to-end production process for final mesenchymal stromal cell composition or the IMP (Investigational Medicinal Product) including proper culturing, manufacturing and cryopreservation and storage of the final cell product in order to achieve consistency in cell yield, superior cell viability, good cell recovery, improved cell proliferation, low HLA-DR expression, purity and potency with reduction in culture duration and reduction in manipulations at the clinical trials site.

The instant disclosure provides a method for culturing of MSCs in a manner so that the no. of cells initially cultured grow multi-fold, thereby leading to high yield of the MSCs by the time the culturing process is completed. The washing process of the instant disclosure further provides beneficial effects on the MSCs obtained by said culturing and reduces or completely eliminates the cells of residual BSA levels that may be present due to use of bovine components during the process of culturing. Subsequently, these MSCs are either stored along with a suitable cryopreservation solution known in the art or are formulated using various vehicles/excipients. In an embodiment of the present disclosure, the term cell composition refers to a final composition comprising allogeneic mesenchymal stromal cells obtained by the above mentioned culturing process of the instant disclosure along with appropriate vehicles/excipients. It is also referred to as Investigational medicinal product/ Investigational product. As used herein, the terms "Investigational Medicinal Product (IMP) or Investigational product (IP) or therapeutic composition or composition" all means a composition comprising mainly of bone marrow derived allogenic Mesenchymal stromal cells, preferably Mesenchymal stem cells, with commercially available ready to use cryopreservation solution such as CryoStor 5 and CryoStor10, or with in-house prepared cryopreservation solution comprising of Human Serum Albumin, Plasmalyte A and Dimethyl sulfoxide (DMSO). Further, the allogeneic MSCs provided in the composition or IMP or IP of the present disclosure, may comprise cells from a single donor or multiple donors. In cases where the cells are provided by more than one donor, the cells are pooled. Preferably, the cell composition/IMP/IP comprises pooled MSCs in CS5.

In an embodiment, clinical and/or therapeutic applications of the MSCs cultured by the culturing process of the instant disclosure, or by the IMP formulated from the MSCs of the instant disclosure, include but not limited to Dilated Cardiomyopathy, Osteoarthritis (OA), Chronic Obstructive Pulmonary Disease (COPD), Ischemic Cardiomyopathy (ICM), Critical Limb Ischemia (CLI), Liver Cirrhosis (LC), Diabetes Mellitus and Acute Myocardial Infarction (AMI).

In another embodiment of the present disclosure, the process of culturing of the MSCs provided by the instant disclosure is scalable and the results are reproducible regardless of the scale at which the culturing is performed. For the ease of explanation and experimentation, the values provided within the scope of the instant disclosure are relative to cell containers or cell stacks which have a surface area ranging from about 600 to 700 cm². The seeding density employed in the present disclosure is in the range of about 1000 cells/ cm² about 7000 cells/ cm². The term 'stack' within the purview of the instant disclosure relates to cells containers having the aforementioned surface area, and wherein the cells are cultured at a seeding density of about 1000 cells/ cm² about 7000 cells/ cm2. Further, the culture media provided to each stage of the culturing is at a concentration ranging from about 0.15 to about 0.35 ml/ cm², and thus the amount of media is provided according to the surface area used for culturing of the cells, regardless of the amount of cells so cultured. A person skilled in the art will therefore understand, that in order to scale-up the process of the instant disclosure, although the containers or stack, and respective surface areas may change, as long as the seeding density and the relative no. of cells and respective amounts of culture medium as disclosed in the instant disclosure coincide with such scaling up, the results obtained will be the same.

The present disclosure presents a method of large scale expansion for therapeutic application of bone marrow derived MSCs. It also discloses essential method of preserving/storing of freshly harvested Mesenchymal Stromal cells (MSCs/ frozen-thawed MSCs for/before transplantation in optimal conditions so as to maintain their viability and multipotentiality for specified durations. The present disclosure also relates to a composition comprising Mesenchymal Stromal/Stem cells (MSCs) along with CS5, CS10 or Human Serum Albumin, Plasmalyte A and Dimethyl sulfoxide (DMSO) and other pharmaceutically acceptable excipients used in cell composition for clinical applications.

In another embodiment of the present disclosure, the stromal cells isolated from the bone marrow are cultured in a complete cell culture medium. The complete cell culture medium (Table 2) comprises of DMEM-KO, FBS, Glutamine, Pen-Strep and basic Fibroblast growth Factor (bFGF). Mononuclear cells (MNC) from bone marrow are obtained by any of the methods established in the prior art.

**Table 1: The table 1 depicts the seeding density of the stromal cells at various passages during the large scale expansion:**

| **Passages** | **Seeding Density:** |
|---|---|
| P0 (MNC) | 40-50 Million cells |
| P1-P5 | 1000-7000cells/cm² |

In yet another embodiment of the present disclosure, the seeding density at P1 is preferably about 6666 cells/cm² and the seeding density at P2-P5 is preferably about 1000 cells/cm². The cells are cultured with intermittent medium change once the cells are confluent, after which they are harvested.

The process of cell expansion in general comprises steps of cell culturing, cell harvesting and thawing at each passage. These steps are carried out after aspirating the cells from biological source such as bone marrow and thereafter diluting in cell culture media as iterated above. The general process of diluting is followed by culturing, harvesting and cryopreservation as disclosed below up to passage 3:
**Cell Culturing:** The cell culturing process comprises seeding cells in culture plates/cell stacks/flasks with complete culture medium and their expansion for certain time period of about 10-14 days. The step of media change is performed at intermittent frequency by replacement of the spent medium with fresh complete medium. Once the cells achieve 80-85% confluence, the cells are harvested.
**Cell Harvesting:** Cell harvesting step comprises removing the spent medium and then washing the cells with DPBS, and thereafter treating the cells with 0.25% trypsin/EDTA for a couple of minutes (2-3 mins). This is referred to as trypsinization. The trypsinized cells are neutralized or in other words, the action/effect of trypsin on the cell is neutralized using neutralizing media. Neutralization medium comprises Dulbecco's Modified Eagle Medium-Knock Out [DMEM- KO], about 10% Fetal Bovine Serum [FBS], about 50 to about 100U/ml penicillin and about 50 to about 100µg/ml streptomycin. After neutralization, the cell suspension in the neutralizing medium is centrifuged to obtain the MSC pellet, which is suspended into culture media for further passage. At this stage, the cells are said to be in Passage 1. On the contrary, the MSC pellet can be cryopreserved using a cryopreservation medium. The cryopreservation medium comprises of Fetal Bovine Serum (FBS) ranging from about 85% to about 95% and Dimethyl Sulphoxide (DMSO) ranging from about 5% to about 15%.

In an embodiment of the present disclosure, the cryopreservation medium is cryostro-5 or cryostro-10 or a combination thereof.

**Thawing:** The cell vial/bag from the cryostor in liquid nitrogen is retrieved and revived in a water bath at 37°C for 3-4 min.

**Table 2: Constituents of various media employing in the present cell culturing process**

| **Medium** | **Composition** |
|---|---|
| Complete cell culture medium devoid of bFGF | DMEM-KO, FBS, glutamine and penicillin-streptomycin |
| Complete cell culture medium with bFGF | DMEM-KO, FBS, Glutamine, Pen-Strep and basic Fibroblast growth Factor (bFGF). |
| Neutralizing medium | DMEM- KO, Fetal Bovine Serum [FBS] Pen-Strep |
| Cryopreservation/freezing medium | CryoStor^{®} family of commercial available animal protein-free, defined cryopreservation medium from Biolife Solutions or |
| | CryoStor^{®}5 (CS5) is an optimized freeze media pre-formulated with 5% DMSO) or |
| | CryoStor^{®}10 (CS10) is an optimized freeze media pre-formulated with 10% DMSO. or |
| | In house prepared cryopreservation solution: Human Serum Albumin, Plasmalyte A and Dimethyl sulfoxide (DMSO) or |
| | FBS and DMSO |

According to the present disclosure, bone marrow is isolated separately from multiple healthy donors wherein the cell population isolated from the bone marrow at passage 0 (P0) is a heterogeneous population of cells. The P0 cells are passaged to obtain P1 cells mainly comprising of Mesenchymal stromal cells. The P1 cells are passaged to obtain the P2, which are further passaged to obtain P3 cells. At each passage, few vials are cryopreserved. In an embodiment the cells from P1 of multiple donors are pooled and passaged to P2 in equal proportion to obtain a pooled sample which is further passaged to P3.

In still another embodiment of the present disclosure, the cells could be directly passaged without pooling to Passage 2 followed by Passage 3(P3).

The cells harvested at passage 3 are either cryopreserved or can be further passaged to P4.

The cryopreserved P3 cells are thawed and seeded into complete cell culture medium. The cell are further cultured and harvested to arrive at P4. Culturing and cell harvesting is carried out as per the above disclosed process of cell culturing and harvesting or even any known process in art can be employed. The P4 cells are thawed and cultured in large scale using the large scale process as described further to obtain cell composition comprising potent, viable, MSCs free of particles/impurities etc.

Optimization of the process minimizes the cost of the goods (COGs) and, therefore, improves overall commercial viability of the product. However, optimization of the IMP manufacturing processes must be based on industry requirements and/or standards of GMP practice. Development and manufacturing of a therapeutic stromal/stem cell product requires extensive quality control (QC) to ensure the purity of the cells. The present disclosure relates to culturing and expanding cells followed by harvesting, washing with DPBS and centrifuging to obtain viable and high cell yield with reduce the BSA levels in the final product which facilitates further optimization of the manufacturing process. Further, apart from the process described above for culturing and harvesting of cells to arrive at cells in Passage 3, processes used conventionally may also be employed within the purview of the instant disclosure. Thus, a person skilled in the art will recognize the optimum process of culturing for arriving at the cells at P3 and proceed to large scale production as disclosed below.

### Large Scale Production:

The large scale production in the present disclosure begins with preparing complete culture medium in a correct proportion of required components to achieve an optimal cell culture medium in order to manufacture cell of clinical quality consistently in large scale. bFGF is one of the important components in the cell culture medium and its concentration is very critical in the process for cell proliferation and HLA-DR expression. In order to provide the precise uniform concentration of bFGF and to avoid any volumetric errors, a bFGF master mix is prepared which is mixed with the culture medium along with other components to obtain the complete culture medium. In the conventional processes, if bFGF is present, it is added to each culture flask/vessel thus leading to a large variation in the cell quality.

In the instant process, the critical implications of media preparation, seed preparation and media feeding cycle during large scale expansion maximizes the efficiency and consistency of the IMP. Preparation of bFGF master mix avoids volumetric error and provides uniform distribution of bFGF to media. Further, feeding bFGF media to all the cell stacks improves the consistency in growth characteristics and cell yield. Hence bFGF master mix provides significant contribution towards maintaining the consistency in production process, with respect to higher cell yield and quality of cells.

The next important step is to prepare seed master mix which provides uniform cell distribution to all the Cell Stacks thus enhancing the uniform proliferation of MSCs and achieving uniform confluency throughout the Cell Stacks during the large scale expansion.

Another critical aspect for large scale expansion is feeding schedule /feeding cycle when culture is ongoing. The feeding cycle frequency is defined by the cell confluency in the culture vessel. This ensures high cell proliferation, consistency in cell yield and reduces the culture duration.

Depending on the number of cells required for IMP dose formulation, the specific number of P3 vials are optionally revived (when cryopreserved) and counted.

The culture media provided to each stage of the culturing is at a concentration ranging from about 0.15 to about 0.35 ml/ cm², and thus the amount of media is provided according to the surface area used for culturing of the cells, regardless of the amount of cells so cultured.

The cells at passage 3 are then expanded to passage 4 by seeding the cells at a seeding density ranging from about 1000 cells/cm² to about 7000 cells/cm², replenishing the cells with about 0.15 to about 0.35 ml/ cm² of the media on day 1 of the culture and further replenishing the cells with about 0.15 to about 0.35 ml/ cm² of the media when a confluency of about 40 to 50% is achieved. The number of the cells at this stage increases by at least about 30 folds when compared to the cell number at the end of passage 3.

Further, these cells are expanded into passage 5 by seeding the cells at a seeding density ranging from about 1000 cells /cm² to about 7000 cells/cm². These cells are replenished with about 0.15 to about 0.35 ml/ cm² of the culture media on the first day of the cultures. Upon achieving a confluency of about 30 to 40%, about 0.15 to about 0.35 ml/ cm² of the culture media is added, without removing the spent medium (media top up). Thereafter, when, the cultures reach a confluency of about 60 to 70%, a step of complete media change is performed, wherein spent media is removed completely from all TCS and 0.15 to about 0.35 ml/ cm² of the culture media is added. The number of the cells at this stage increases by at least about 2000 folds when compared to the cell number initially at the end of passage 3.

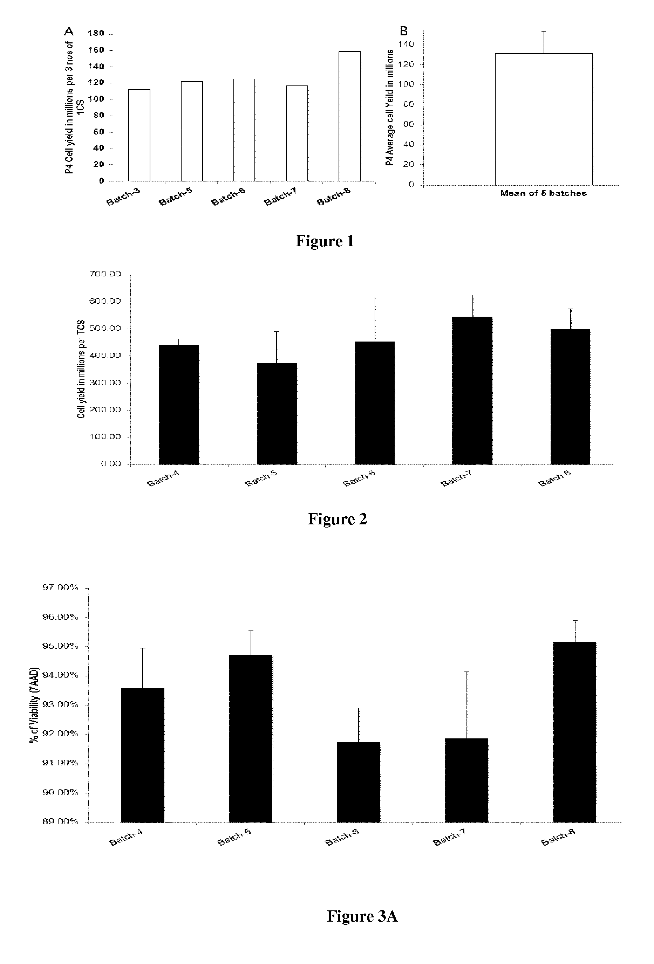

More particularly, as an exemplary embodiment, the cells at passage 3 are expanded to passage 4 by seeding the cells in one cell stack (having a surface area of about 636 cm²) at a seeding density of about 1000 cells/cm², replenishing the cells with about 150 ml of the media on day 1 of the culture and further replenishing the cells with about 150 ml of the media when a confluency of about 40 to 50% is achieved. The yield obtained at this stage is about 25-40 ×10⁶cells per 1CS and the total duration required for this process is about 8 to 9 days (Figure 1A and 1B).

Further, these cells are expanded into passage 5 by seeding the cells in ten cell stacks (having a surface area of about 636 cm X 10 = 6360 cm²), at a seeding density of about 1000 cells /cm² per cell stack. These cells are replenished with about 1.5 L of the media per cell stack on the first day of the cultures. Upon achieving a confluency of about 30 to 40%, 0.5 L of the media is added to each cell stack, without removing the spent medium (media top up). Thereafter, when, the cultures reach a confluency of about 60% to 70%, a step of complete media change is performed, wherein spent media is removed completely from all TCS and about 2L of fresh media is added to each cell stack. The yield obtained at this stage is about 450-600 × 10⁶ cells per 10 cell stacks and the total duration required for this process is about 10 to 11 days (Figure 2).

Calculations of the aforementioned culturing can be represented as below:
The volume of 1 cell stack is about 600 cm² to about 700 cm². At a seeding density of about 1000 cells/cm², total number of cells seeded at beginning of passage 4 in 1 cell stack is about 6,00,000 to about 7,00,000 cells or about 0.6 million to about 0.7 million cells. Due to expansion of cells, by the end of passage 4, the number of cells increase to about 25 million cells to about 40 million cells per cell stack.

These cells are then seeded in different cell stacks again at a seeding density of about 1000 cells/cm² for further expansion into passage 5. Since 1 cell stack can accommodate about 0.6 million to about 0.7 million cells, number of cell stacks required to accommodate about 25 million cells to about 40 million cells is about 40 to about 70 cell stacks. Now as mentioned above, since expansion of cells from end of passage 4 to end of passage 5 provides a cell yield of about 450-600 x 10⁶ cells per 10 cell stacks, i.e., 45 to 60 million cells per cell stack, such expansion for each of about 40 to about 70 cell stacks will lead to total number of cells in the range of 1800 million cells to about 4200 million cells. Thus, initially from about 0.6 million to about 0.7 million cells, the expansion in passage 4 and passage 5 leads to total number of cells in the range of about 1800 million cells to about 4200 million cells, thereby increasing the cells by at least 2000 folds.

However, the cells may be seeded at a density ranging from about 1000 cells/cm² to about 7000 cells/cm². In case the seeding density is increased, the duration of culturing shall accordingly vary and indeed be reduced. Further, the media consumption shall also be less, as lesser number of cell stacks will be required to seed more number of cells.

The aforementioned process of media feeding plays an important role in large scale expansion as media top up at about 30% to 40 % confluency improves the proliferation of the MSCs. Further complete media change (CMC) at about 60% to 70% confluency enhances the proliferation without compromising the viability and quality of large scale cultures. Further CMC improves the consistency in cell yield and reduces the culture duration.

The cells harvested from large scale expansion process are then subjected to a wash process to remove all the impurity /particles especially BSA which is found in the harvested cells due to use of FBS during the cell culture. The cell composition used for clinical and/or therapeutic applications needs to be free of all xeno components, and the levels of BSA always needs to be less than 50ng for each of such compositions, regardless of the number of cells or volume of such composition. The wash process followed herein involves the following steps:
1. The cells are first centrifuged at about 1200 rpm to about 1800rpm for about 10 minutes. The cells are then washed with about 200 ml of DPBS/10 cell stack and re-centrifuged at about 1200 rpm to about 1800 rpm for about 10 minutes.
2. In the second wash, about 70 ml to about 90 ml of the washing buffer-DPBS is added per 10 cell stack followed by pooling of the cell samples from all cell stacks.
3. The pooled cells are again centrifuged at about 1200 to about 1800 rpm for about 5 minutes to about 7 minutes.
4. Finally cell pellet will be formulated in CS5 at about 25M cells/ml and frozen at 2ml, 4ml and 8ml volume.

The washing steps as indicated above to minimize the BSA levels (i.e. < 50ng) and maintain good viability and reduce cell loss in the production process of the final product, which is a composition comprising mainly of bone marrow derived allogenic Mesenchymal stromal cells, CS5, or CS10 or Human Serum Albumin, Plasmalyte A and Dimethyl sulfoxide (DMSO). It is important to note here that as indicated previously, after the culturing and washing of the cells as per the protocol of the instant disclosure, the cells are formulated as IMP or cell compositions as per the required dosage for clinical and/or therapeutic applications. The levels of BSA in any such IMP or cell compositions are always maintained below 50ng regardless of the number of cells or volume of the composition.

It is known in the art that cell composition is cryopreserved using freezing mix/cryopreservation mix comprising Plasmalyte-A, DMSO and Human Serum Albumin (HSA). On clinical requirement the cryopreserved cells are thawed and reconstituted as per the requirement of dosage for clinical administration. The process of reconstitution is carried out in clean room having highly regulated environment and also requires skilled person to carry out the process which is very expensive and time consuming.

On the contrary, the present disclosure discloses a method by which the cell composition can be easily administered without requiring a process of reconstitution at the site of clinical administration. This is carried out by storing the MSCs obtained by the culturing process of the instant disclosure directly in a freezing mixture which has relatively lower amounts of DMSO. One such suitable freezing mixture is Cryostor 5 (CS5). The IMP of the instant disclosure is thus obtained by preserving the MSCs directly in the low DMSO cryopreservation media preferably Cryostor 5 (CS5) in Crystal Zenith vials (CZ vials). This step is not mandatory to the process of preparing the IMP or arriving at the required number of cells through culturing steps of the instant disclosure, but is important for final delivery of the cell product in the market for clinical and/or therapeutic use. In this step, the high freezing density of cells is about 5-25 million cells per ml of the freezing mixture.

According to the conventionally known processes, the cells normally are cryopreserved for any duration of time at minus (-) 196°C, whereas in the present disclosure, high cell density in low volume (of about 5-25 million cells per ml of the cryopreservation mixture/freezing mixture) is cryopreserved at minus (-) 80°C for shorter durations and (-) 196°C for longer durations. Stability tests are conducted using direct stability study to check for the product shelf life.

Further, the present disclosure also relates to a concept called as 'direct stability' to improve the shelf life period of IMP, wherein the MSCs are frozen at 25Million cells per ml of CryoStor 5 in CZ vials and stored directly at minus (-) 80°C. This method of storage of the cells does not involve any exposure to liquid nitrogen at -196°C as conventionally done. This procedure provides for superior short term storage of MSCs for a period of about 1 month at -80°C and is free from hazardous and costlier liquid nitrogen based cryostore procedures. On the other hand, if IMP needs to be stored for a longer period, such as beyond few weeks or months, preferably beyond 1 month, the cells obtained from the culturing and washing process of the present disclosure is cryopreserved at high cell density of about 5 million to about 25 million cells per ml of the cryopreservation solution, preferably CS5, at (-) 196°C. The combination of high freezing density and storage at minus (-) 196°C does not impact the cell viability and cell recovery on thawing.

The combination of high freezing density and CS5 in storage at minus (-) 80°C or (-) 196°C does not impact the cell viability and cell recovery on thawing. Thus, the cells obtained from the process employed above to prepare various doses of IMP after DPBS washes are stored at high freezing density in CS5 at minus (-) 80°C or (-) 196°C, as per the requirements for optimum results.

The present disclosure thus provides for enhanced final composition for various clinical and/or therapeutic applications by applying multiple optimizations in the overall culturing and storing processes of MSCs. The said optimizations of the present disclosure are summarized as below:

### Culturing stage:

- The initial relatively small number of pre-cultured MSCs (pre-cultured to a predetermined passage such as passage 3) is subjected to expansion using the culture medium having bFGF. The said culture medium is prepared by master mixing of components which eliminates volumetric errors and inconsistencies of the components, particularly bFGF, as the culturing is carried out simultaneously in multiple containers (cell stacks) to expand the cells rapidly. Conventional mixing of components does not provide equal and consistent volumes of bFGF in the media, thereby leading to differential growth in cells in different containers. The master mixing therefore provides advantage to the overall culturing process.
- Another important aspect of the process of expansion is to seed the cell suspension in a manner which again avoids/eliminates volumetric errors and inconsistencies of the components and variations from batch to batch or containers to containers.
- Upon master mixing of the culture medium and following seeding protocol of the instant disclosure, expansion of cells take place, from one passage to another and wherein the culture media is replenished or topped based on confluency of the cells, rather than the no. of days into culturing, as previously known.
- All the aforementioned aspects in totality lead to high yield of MSCs, leading to multi-fold increase in the no. of cells from the initial number; along with ensuring that the MSCs so obtained are high in quality, consistency, immunosuppressive and immunopotent properties, and low in negative markers such as HLA-DR.

### Storing stage:

- Once the cells are obtained by the aforementioned culturing process, they may be either formulated for storage or formulated for instant administration for clinical and/or therapeutic purposes.
- Conventionally, for purposes of storing the MSCs, a cryopreservation solution is most commonly used for freezing the cells. The MSCs are frozen in cryopreservation solution and stored in liquid nitrogen, and as and when required, thawed, reconstituted and used for clinical and/or therapeutic purposes. It is important to note that usually, such cryopreservation solution have high concentrations of DMSO, which may cause toxicity to humans after administration. Hence, it is necessary to reduce the toxicity by diluting or washing or completely removing the DMSO from cryopreserved cells before the cells can be used for clinical and/or therapeutic purposes. Further, since the freezing and storing from previously known methods requires use of liquid nitrogen, the process requires clean room facility and skilled person (as liquid nitrogen is hazardous, it needs to be handled carefully), and costly apparatus which is a challenge. Further, this would also make it virtually impossible for storing the cells and transporting them at the same time, thereby making the entire process inconvenient, tedious and challenging.

- The present disclosure avoids/eliminates the requirements of reconstitution by freezing the cells at high freezing densities using cryopreservation mixture which has less amounts of DMSO, and storage at -80 degree Celsius, which allows retention of stability, viability, immuno-properties and post-thaw viability and recovery of the cells. This process of freezing the cells is optimum for short term storage of the cells.
- Thus, the present disclosure provides culturing of cells as per the protocol disclosed above to obtain high yield of MSCs. These MSCs will then be cryopreserved in Cryostor 5 (CS5) at a high freezing density ranging from about 5 million to about 25 million cells per ml of CS5, and shall be frozen preferably in CZ vials for a time period of about 1 month at a temperature ranging from about -70 degrees Celsius to about -80 degrees Celsius. On the contrary, for longer storage, the same cell density and cryopreservation solution is used to store cells at -196 degree Celsius.
- As and when the cells are required for clinical and/or therapeutic applications, the frozen vials are removed from the storing temperature of -80 degrees Celsius or -196 degrees Celsius, as the case may be, and only thawed. No reconstitution of cells is required, and the cells along with CS5 can directly be administered to a subject in need thereof, for clinical and/or therapeutic applications.

### Additional step of washing:

- The MSCs which are cultured using any culture media comprising any bovine component are usually bound to have a certain amount of the bovine component present with them, even at the final stage of culturing. In other words, if culturing of MSCs at any stage involves use of any bovine components, the cells harvesting at the final stage will have some remnants of the bovine components associated with the cells. This makes the cells unfit for administration in humans, and therefore proper washing and removal of such bovine components is required.
- In the culturing process of the present disclosure, fetal bovine serum is used during the process. The FBS contain bovine serum albumin (BSA), and therefore, upon harvesting of the cells, it is essential that such BSA is removed. Wash processes which are previously known involve multiple centrifugation and wash processes, which not only have a risk of reducing the cell loss and viability due to multiple centrifugation but also are not capable of reducing the BSA amounts associated with the MSCs to the desired levels. The new wash process and centrifugation of the instant disclosure has resulted in reducing the cell loss and increasing viability during the multiple washes and centrifugation thus reducing the cell loss and increasing the cell viability of the final product.
- The present disclosure thus provides a wash process which overcomes the said drawbacks of the previously known techniques and allows further reduction of the BSA levels, with lesser cycles of centrifugation.

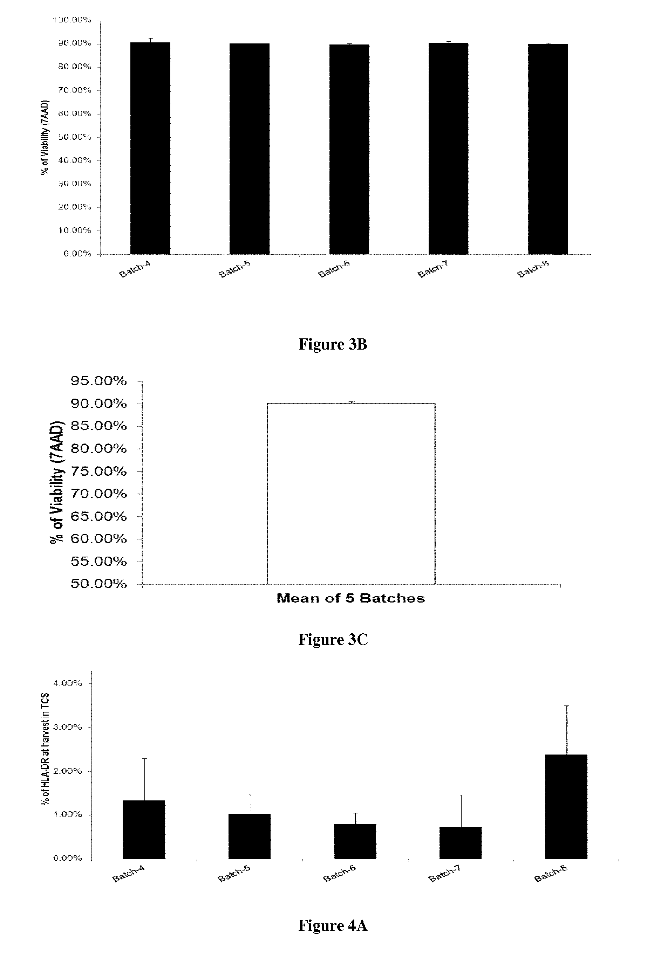

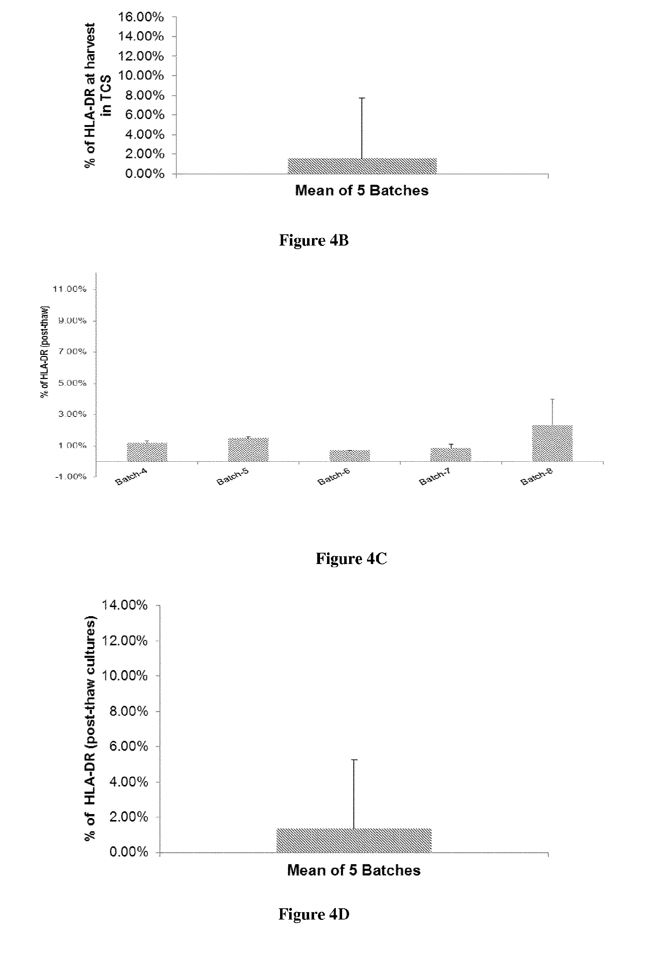

The advantages of the process of the instant disclosure are thus as follows:
i. Batch to batch consistency in all ten Cell Stacks with respect to cell number, viability and HLA-DR expression
   a. Increase in cell yield
   b. Increase in cell viability (Figures 3A to 3C)
   c. Low expression of HLA-DR (i.e. <5%) (Figures 4A to 4D)
ii. Reduced cell loss during production process
iii. Reduced impurities in the final product
iv. Reduction in manipulations at the clinical trials site. In other words, manufacturing a bench to bedside product to be administered to the patient without having to reconstitute/dilute the cell composition.
v. The final product is ready to use at the bench side like a pharmaceutical product

The present disclosure is further elaborated by the following examples and figures. However, these examples should not be construed to limit the scope of the disclosure.

### EXAMPLE 1:

### Isolation and cell culture of Bone Marrow derived MSCs up to Passage 3 (P3):

About 60-70ml bone marrow from healthy volunteers in the age range of 20-40 years is aspirated from the upper posterior of the iliac crest using a heparinized syringe, under general anaesthesia, in a general operating theatre, after obtaining informed consent, and is collected into individual blood bags. 20 ml syringe is used to transfer the sample through the cell strainer (100µm) to remove bone spicules, blood clots and cell aggregates and collected into a centrifuge tube. Before taking the bone marrow, the volunteers are screened for human immunodeficiency virus (HIV1), hepatitis B (HBV), hepatitis C (HCV) and cytomegalovirus (CMV) as a mandatory screening test.

Bone marrow processing and subsequent cultures are carried out in a current good manufacturing practice (cGMP). Briefly, bone marrow is diluted (1: 1) with complete culture media comprising Dulbecco's modified Eagle's medium knock-out (DMEM-KO) 80%, Fetal Bovine Serum (10%), 1M mol Glutamine and 50 to 100U/ml penicillin and 50 to 100µg/ml streptomycin and centrifuged at about 1200 to about 1800 r.p.m. for about 10 minutes to about 20 minutes to remove the anticoagulant. The complete culture media used for diluting above is devoid of bFGF. The supernatant is removed and the bone marrow is again diluted with complete culture media without bFGF. The bone marrow derived mononuclear cells (MNCs) are separated by the density gradient methods using Lymphoprep in 50 ml centrifuge tubes (Falcon, BectonDickinson). This process involved taking another centrifuge tube lymphoprep [density gradient solution] and adding double the volume of diluted bone marrow and centrifuging at about 1200 rpm to 1800 rpm for about 10 minutes to 20 minutes at room temperature, wherein the room temperature is 20°C to 25°C. Bone marrow MNCs accumulated on the buffy layer interface are isolated and washed again with complete culture media devoid of bFGF. Isolated mononuclear cells at passage 0(P0) are plated into T-75 culture flasks (Falcon, BectonDickinson) and cultured in DMEM-KO supplemented with 10% fetal bovine serum (HyClone), 200 m M glutamax (Gibco-Invitrogen), 50 to 100U/ml penicillin and 50 to 100µg/ml streptomycin_and incubated at 37 °C and 5% humidified CO₂. The non-adherent cells are removed after 48 h and first complete change of culture medium is carried out. Subsequently the medium is replenished every 48 h. Frequent media change ensures that predominantly the MSCs are attached to the surface because of their unique plastic adherence property. Upon desired confluency of about 80%-85%, aspirate out the complete media and wash the cells twice with Dulbecco's Phosphate Buffer Saline (DPBS). Add about 1-2 ml of trypsin per T-75 flask and incubate at 37°C for about 2 minutes to 3 minutes. The action of trypsin is neutralized with neutralization media comprising DMEM KO, 10% FBS and Pen-Strep [50 to 100U/ml penicillin and 50 to 100µg/ml streptomycin] and the neutralized sample is collected and centrifuge at about 1200 rpm to 1500 rpm for about 10 minutes to 20 minutes at room temperature. To the pellet add complete media, followed by cell counting. Thereafter, freeze MSCs in vials at concentration of about 1 million per ml using the freezing media comprising of about 85% to 95% FBS and about 5% to 15% DMSO, this is referred to as Passage 0 (P0) Cells (i.e.1-2 vials). The rest of the cells are cultured/expanded in cell stacks at a seeding density of about 6,666 cells per sq.cm with complete media change on 7^{th} or 8^{th} day (culture age) and the cells are harvested with 0.25% trypsin/EDTA (Gibco-Invitrogen) between 14 to 18th day. Trypsinized cells are neutralized with neutralization media comprising DMEM KO, 10% FBS and Pen-Strep [50 to 100U/ml penicillin and 50 to 100µg/ml streptomycin] and collected in a centrifugation tube for centrifugation at about 1200 rpm to 1500 rpm for about 10 minutes to 20 minutes. Pellet is resuspended in complete media to assess cell count. Freeze harvested cells in vials at a concentration of about 1 million cells to 3 million cells per ml in the freezing media comprising of about 85% to 95% FBS and about 5% to 15% DMSO this is referred as Passage 1 (P1) cells.
MSCs from Passage 1 is re-plated in a single one-cell stack with a seeding density of 6,666 cells per sq.cm and cultured in DMEM-KO supplemented with 10% fetal bovine serum (HyClone), 200 m M glutamax (Gibco-Invitrogen), 50 to 100U/ml penicillin and 50 to 100µg/ml streptomycin_and incubated at 37 °C and 5% humidified CO₂. Briefly, the process is as follows: One vial each of P1 cells is taken, and counted and optionally pooled in equal proposition of each donor, in cases where the initial bone marrow was aspirated from more than one donor. After counting and optional pooling of donor cells, cells are checked for viability; and viable cells are plated into 2 -10 chambers as per the cell counts obtained. Thereafter, transfer the culture chambers to a 5% CO₂ incubator at about 37°C. After every 48 -72 hours observe the cell stacks under microscope and take two representative microphotographs. Replenish the complete culture medium every 7th to 8th day with the freshly prepared complete media comprising DMEM-KO, FBS, Glutamine, Pen-Strep and basic Fibroblast growth Factor (bFGF). The cells are cultured until the cells in the chambers are about 80%-85% confluent.

Once Passage 1 cultures are confluent they are further expanded up to passage 3 by harvesting confluent cultures and seeding at seeding density of 1000 cells/cm² and cultured in DMEM-KO supplemented with 10% fetal bovine serum (HyClone), 200 mM glutamax (Gibco-Invitrogen), 50 to 100U/ml penicillin and 50 to 100µg/ml streptomycin_and additionally adding growth factor of 2ng/ml basic fibroblast growth factor (bFGF) and incubated at 37 °C and 5% humidified CO₂. For large scale production, MSCs at P3 are plated onto one 10-cell stack (Corning Life Sciences). Briefly, the process is as follows: When the P1 cells attain about 80% to 85% confluency, the complete media is aspirated out and the cell stacks are washed twice with DPBS. After washing trypsin is added, and the cells are incubated at about 37°C for about 4 minutes to 5 minutes, thereafter neutralize them with neutralization media. The neutralized sample is collected and centrifuged at about 1200 rpm to 1800 rpm for about 10 minutes to 20 minutes at room temperature. To the pellet hence obtained, complete media is added and the cells counted. Thereafter culture/expand the cells in the cell stacks for another passage, harvest the cells and centrifuge. To the pellet obtained, complete media is added and the cells are counted. The vials are frozen at about -196°C, such that each vial contains about 1-3 million MSCs in freezing mix comprising about 85% to 95% FBS and about 5% to 15% DMSO. This forms the cells at Passage 3 (P3) and thereafter used for future large scale expansion.

### PREPARATION OF IMP COMPOSITION

### Process For Preparing IMP Or The Final Composition Of Bone Marrow Derived Stromal Cells After Passage 3

The process for preparing IMP or the final composition of bone marrow derived MSCs begins by initially culturing the cells beyond passage 3 to obtain high yield of MSCs which possess high quality and immune properties. The process after Passage 3 begins by initially preparing the bFGF master mix and the culture medium comprising this master mix for uniform distribution of said bFGF without any volumetric error. Thereafter, the process comprises preparation of seed master mix, followed by seeding of the cells, culturing and changing of media as per a defined schedule for further expansion of cells. The expanded culture is then washed to remove BSA, and the cells proceed for preparation of the final composition or the IMP, as per the required clinical and/or therapeutic applications. The process is further defined sequentially below.

### 1. Preparation of culture Medium comprising bFGF Master Mix for use in culturing and processing of cells after Passage 3:

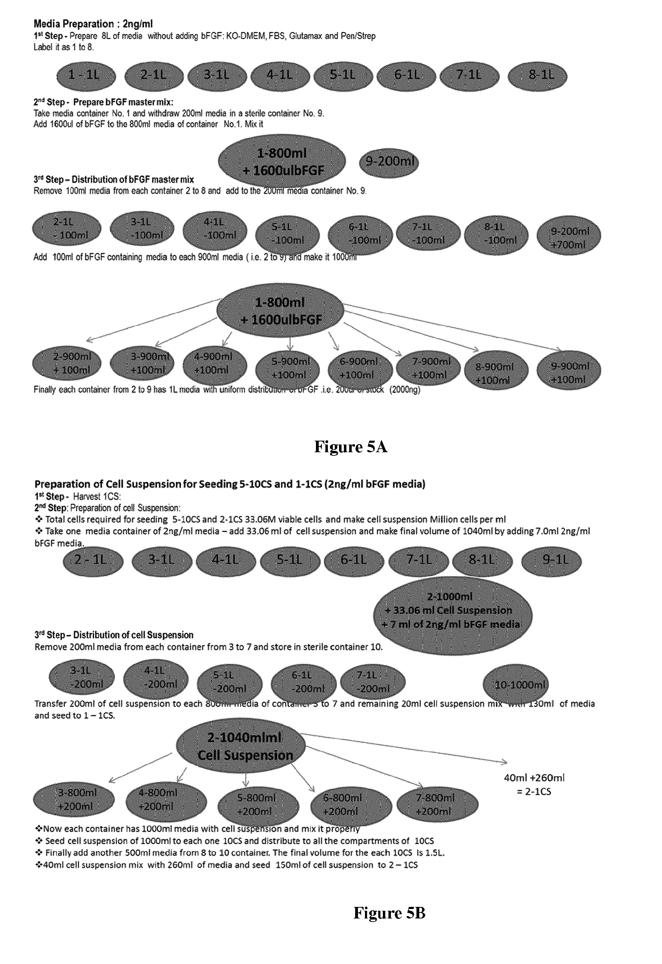

Preparation of bFGF master mix avoids volumetric error and provides uniform distribution of bFGF to complete cell culture medium used for cell culturing/expansion. Thus prepared complete cell culture medium when added to the cell stacks ensures that uniform concentration of bFGF is provided to the cells. This process (Figure 5A) improves the consistency in growth characteristics and cell yield in all the ten layer stacks. Hence bFGF master mix provides significant contribution towards maintaining the consistency in production process, higher cell yield and quality of cells.

### Preparation of culture media having 2ng/ml of bFGF:

1. 8L of media comprising KO-DMEM, FBS, Glutamax and Pen/Strep but devoid of bFGF is prepared and dispensed into eight 1L Steri cups and labelled from 1 to 8.
2. From the media container No. 1, 200 ml media is withdrawn and dispensed into a sterile container labeled No. 9.
3. 1600ul of bFGF (i.e. 16000ng of bFGF) is added to the 800ml media of container No. 1 and mixed well and labeled as bFGF master mix.
4. 100ml media is withdrawn from each container labeled from 2 to 8 and added to the 200 ml media container No. 9, thereby making the total media volume of container No. 9 as 900ml [200ml from container No. 1 + 100 ml each from container Nos. 2 to 8].
5. 100ml of bFGF master mix is taken and and added to each 900 ml media (i.e. container Nos. 2 to 9) and the volume in each of the said containers is thus made upto1000 ml.
   Now each container labeled from 2 to 9 has 1L of media with uniform distribution of bFGF master mix (1000ml of media containing 2000ng of bFGF i.e 2ng/ml of bFGF concentration. This process is represented by Figure 5A.

### 2. Preparation of seed master mix for use in culturing and processing of cells after Passage 3:

Preparation of Seed master mix avoids volumetric error and provides uniform cell distribution to all Cell STACKs. Seed master mix (Figure 5B) provides significant contribution towards process of uniform proliferation and the consistency of in cell yield in all TCS.
1. For each 10CS 6.36 million viable cells are seeded. Total cells required for seeding 5-10CS is 31.8 million viable cells and cell suspension of million cells per ml.
2. One media container that contains 2ng/ml bFGF media is taken, 31.8ml media is removed and 31.8 ml of cell suspension is added. 200ml media is removed from each container with bFGF media labeled from Nos. 3 to 7 and stored separately in sterile container No. 10.
3. 200ml of seed master mix is collected and transferred to each 800ml media of container Nos. 3 to 7.
4. Now each container has 1000ml media with cell suspension. Cell suspension of 1000ml is seeded to each one 10CS and distributed to all the compartments of 10CS.
5. Finally another 500ml media is added to each 10 CS from container Nos. 8 to 10. The final volume for the each 10CS is 1.5L.

This process is represented by Figure 5B.

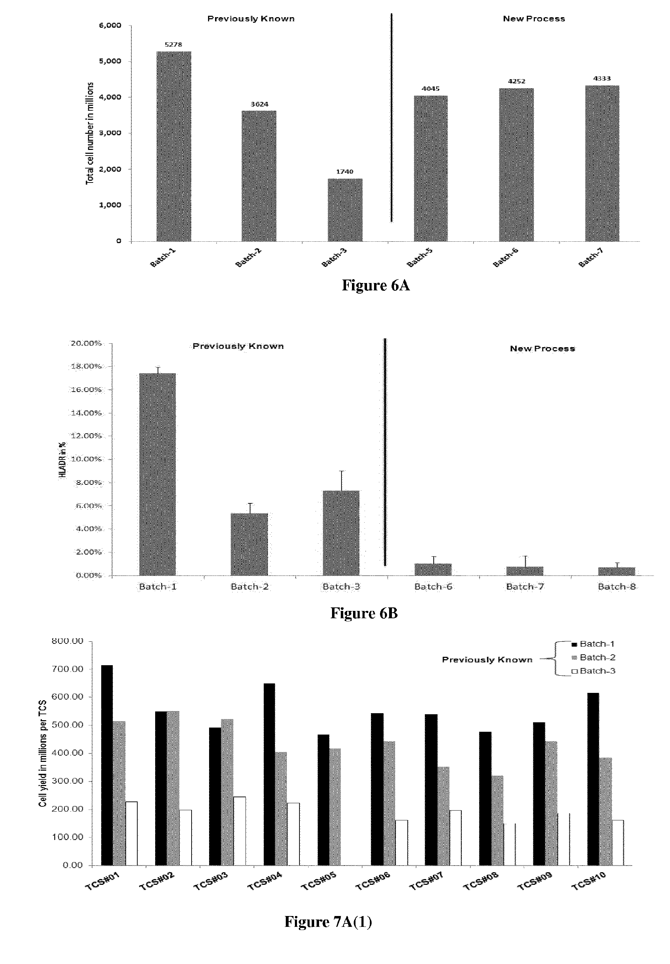

Figures 6A and 6B provides the details of batch consistency data and reduced HLA-DR expression using the bFGF and seed master mix during the large scale production. The figures also show comparison with previously known processes that do not involve such bFGF master mix and seed master mix.

### 3. Cell seeding, culturing and media change schedule:

The cells at passage 3 are expanded to passage 4 by seeding the cells in one cell stack at a seeding density of 1000 cells/cm², replenishing the cells with 150 ml of the media prepared above on day 1 of the culture and further replenishing the cells with 150 ml of the media when a confluency of 40-50% is achieved. The yield obtained at this stage is about 25-40 ×10⁶cells per 1CS and the total duration required for this process is about 8 -9 days.

### 4. Further expansion of cells:

Further, these cells are expanded into passage 5 by seeding the cells in ten cell stacks, at a seeding density of 1000 cells /cm². These cells are replenished with 1.5 L of the media on the first day of the cultures. Upon achieving a confluency of about 30-40%, 0.5 L of the media is added to each cell stack, without removing the spent medium (media top up). When, the cultures reach a confluency of about 60-70%, a step of complete media change is performed, wherein spent media is removed completely from all TCS and 2L of the media is added to each cell stack. The yield obtained at this stage is 450-600 × 10⁶ cells per 10CS and the total duration required for this process is about 09-10 days compared to previously known process of 14 days. Since all the cells obtained at the end of Passage 4 are expanded in different cell stacks ranging from about 40 to about 70 cell stacks at the same seeding density of 0.6 to about 0.7 million cells per cell stack, the total number of cells obtained at the end of passage 5 ranges from about 1800 million cells to about 4200 million cells.

### 5. Washing Process:

These cells are then subjected to a wash process involving the following steps:
- The cells are first centrifuged at about 1200 rpm to about 1800 rpm for about 7 to about 10 minutes. The cells are then washed with about 200 ml of DPBS/ 10 cell stack and re-centrifuged at about 1200 rpm to about 1800 rpm for about 10 minutes.
- In the second wash, about 70 to about 90 ml of the washing buffer-DPBS is added per 10 cell stack followed by pooling of the cell samples.
- The pooled cells are again centrifuged at about 1200 to about 1800 rpm for about 5 to about 7 minutes.

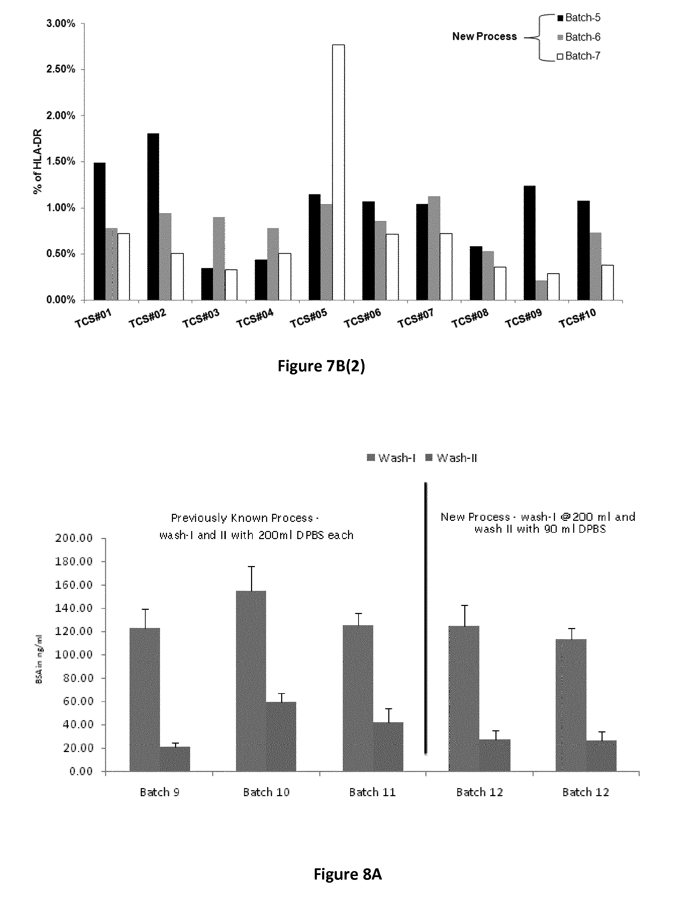

In the complete cell culture medium used for cell expansion after passage 3 (as provided in table 2), FBS is employed as a supplement which is of bovine origin and hence Bovine Serum Albumin (BSA) needs to be removed from the product before clinical application. The washing steps as indicated above reduces the BSA levels (i.e.< 50ng) in the final product, which is a composition comprising mainly of bone marrow derived allogenic MSCs in CS 5. Also the cell composition can be used in combination with CS10 or in combination with Human Serum Albumin, Plasmalyte A and Dimethyl sulfoxide (DMSO). Preferably the final cell composition comprises about 25 to about 200 million mesenchymal stromal cells in about 1 to about 8 ml of CS5. The final composition of the cell density/ml can be 25million cells/ ml of CS5, 50 million cell/2ml of CS5; 100 million in 4 ml of CS5 and/or 200 million cells in 8 ml of CS5. The Figure 8A clearly shows reduction of BSA in the IMP following the above disclosed wash procedure.

*Final products delivery and storage:* The IMP thus obtained is cryopreserved in the formulation media Cryostor 5 (CS5) in Crystal Zenith vials (CZ vials). It can be also cryopreserved in a bag/vial and in any other cryopreservation medium used for cell preservation. This step is not mandatory to the process of preparing the IMP but is important for final delivery. In this step, the high freezing density of cells is 5-25 million cells per ml of the freezing mixture, and the MSCs are directly stored at minus (-) 80°C. This method of storage of the cells, is ideal for shorter duration, and does not involve any exposure to liquid nitrogen at -196°C. This process ensures superior short term storage of the cells, including high cell viability and cell recovery on thawing.

On the other hand, if IMP needs to be stored for a longer period, such as beyond few weeks or months, preferably beyond 1 month, the cells obtained from the culturing and washing process of the present disclosure is cryopreserved at high cell density of about 5 million to about 25 million cells per ml of the cryopreservation solution at (-) 196°C. The combination of high freezing density and storage at minus (-) 196°C does not impact the cell viability and cell recovery on thawing.

The above procedure is mainly related to the final delivery to the market as a ready to use bedside product. The main challenge in cell based product is to deliver the cell based product in such a way that it can be used without any further manipulation. According to the conventional procedures, cell product stored in liquid nitrogen is thawed and processed for reconstitution before being administered to patient. This process requires clean room facility and skilled person which is a challenge for final market delivery. Use of liquid nitrogen for storage and transportation of the cell product is an expensive method and hence the present procedure of storing and transportation of the cell composition at minus (-) 80°C is cost effective.

Thus the present disclosure provides a procedure for formulating, storing and transporting cell composition, wherein high number of cells is frozen in less volume of cryopreservation solution, wherein the cells are cryopreserved at a cell density of 5-25 million/ml of CryoStor 5 and these are directly stored at (-) 80°C for short duration requirement.

The combination of high freezing density in CS5 and storage at minus (-) 80°C does not diminish the cell viability and recovery. The IMP comprises of 25 million cells to 200 million cells at a freezing concentration of 25 million cells/ml of freezing mixture of CryoStor 5 and stored in CZ vials of 5ml to 10ml packing size at either -80°C or -196°C, as per the required duration of freezing or storing.

### EXAMPLE 2:

### Significance of Feeding cycle - Top-up and Complete media change in large scale expansion based on culture confluency:

Media feeding plays an important role in large scale expansion as media top up at 30 to 40 % confluency improves the proliferation of the MSCs. Further complete media change (CMC) at 60 to 70% confluency enhances the proliferation without compromising on the viability and quality of large scale cultures. Further CMC improves the consistency in cell yield and reduces the culture duration.

### Advantage of improvement

i) Batch to batch consistency in all ten cell stacks with respect to viability, cell number and HLA-DR
ii) Increase in cell yield
iii) Low expression of HLA-DR

### Consistency in HLA-DR expression:

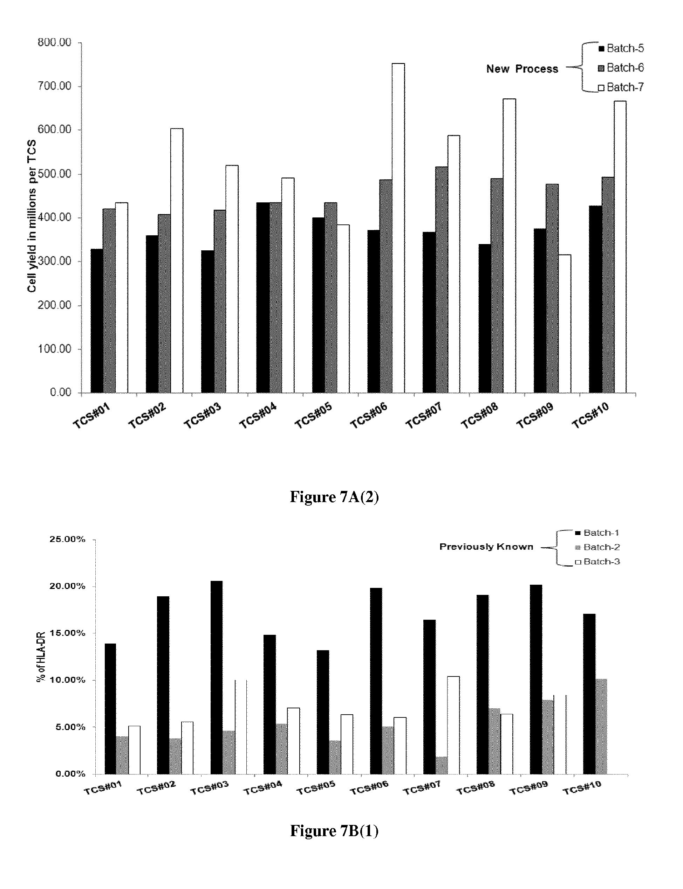

Thus, as shown in Figures 7A and 7B which indicates total cell yield and HLA-DR expression in different production batches, the process of the instant disclosure showcasing a media change based on confluencies, show significantly better results when compared to the previously known process of media change according to the number of days.

### EXAMPLE 3:

### Significance of Washing steps of the present disclosure in in large scale expansion of MSCs:

Washing process to remove the impurities/particles is very critical and fine tuning of the process of wash step is very essential to achieve the required cell quality consistently, and even a slight variation can give varying results. Thus optimization of the manufacturing process for washing and centrifugation minimizes cell loss while reducing the BSA levels and consistently improving post thaw viability of the final product. Many cell therapies are administered intravenously wherein carryover of particulates or intact microcarriers into final products poses a serious safety risk. Extensive optimization and validation for processing large scale cultures to ensure that all particulates are removed from cell suspension increases a system's effective yield. Sequential washing steps with DPBS and centrifugation can facilitate cell separation and help in the removal of particulate materials.

### Advantages of washing process of the instant disclosure

i. Washing steps improve the purity of the IMP.
ii. Reduced cell loss
iii. Washing steps enhance the viability and quality of the final product.

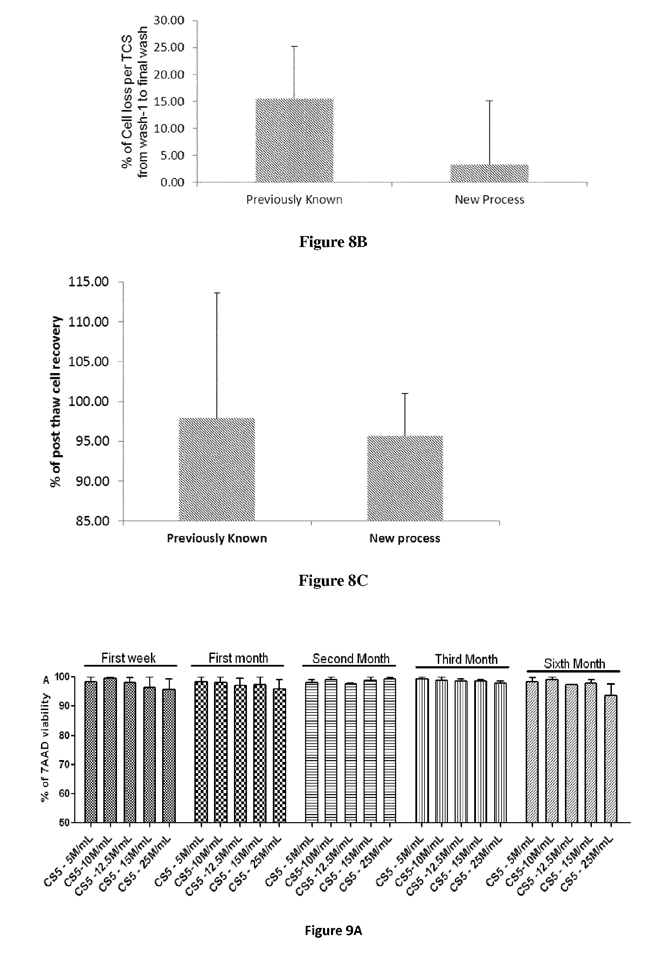

The BSA levels in the final product is minimized in the previously known process by following a wash process involving a first wash with 200 ml of DPBS and centrifugation at 1800 rpm for 10 minutes [wash-I]; followed by a second wash with 200 ml of DPBS and centrifugation at 1800 rpm for 10 minutes [wash-II]. This is compared with the wash process used in the current process of the present disclosure which involves a first wash with 200 ml of DPBS, centrifugation at 1800 rpm for 10 minutes [wash-I]; followed by a second wash with 90 ml of DPBS centrifugation at 1800 rpm for 7 minutes [wash-II]. It is observed that upon following the new wash process of the present disclosure, there is reduction in cell loss (Figure 8B) and the viability of the final product is improved by significant reduction in the levels of BSA (Figure 8A) [as estimated by Bovine Albumin ELISA kit (BSA) kit Catalog No.: 8100; Manufactured by Alpha Diagnostics]. Further, the said washing [wash-II] steps minimize cell loss and improve the post thaw viability/cell recovery of the final product (Figures 8B and 8C). In the previously known process the cell loss was high with low viability thus affecting the cell quality. The present modified/improved wash process is able to reduce the cell loss and cell viability is not affected in the said process and cell quality is improved.

### EXAMPLE 4:

### High freezing density reduces manipulations at the clinical trials site:

The present disclosure aims at developing high freezing density using completely xenofree, serum free freezing media that enhances superior cell viability and multipotency function following thawing. It should allow storage of cell therapy products in a frozen state with high post-thaw viability (years of storage at -196°C). Different freezing concentrations of MSCs were critically evaluated and the concentration of 25M cells per ml of CryoStor 5 was found to be ideal for freezing concentration of final cell therapy product for storage and delivery.

### Viability by 7AAD and cell recovery:

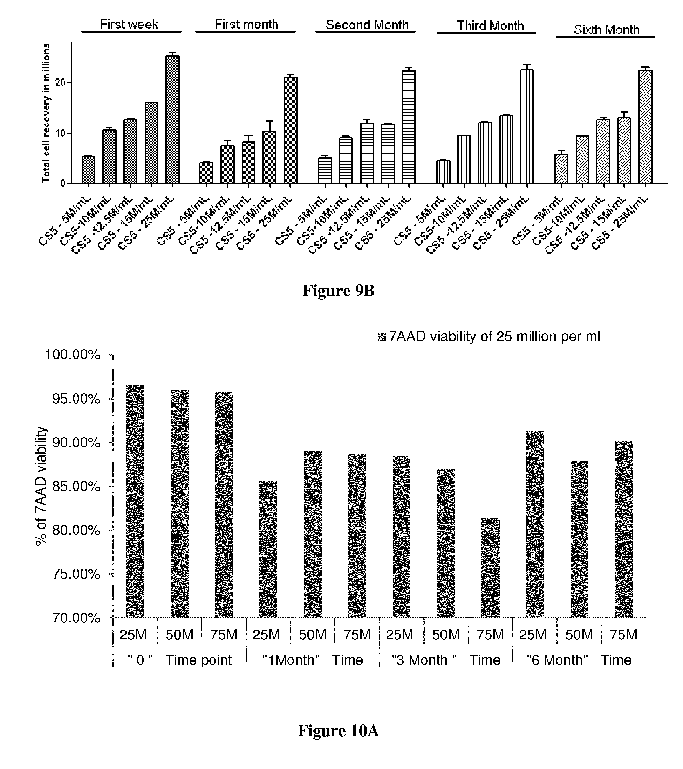

BM-MSCs are frozen using CryoStor 5 in different concentrations of 5, 10, 12.5, 15 and 25 million cells per ml of cryopreservation media CryoStor 5. Post-thaw cell recovery and viability are analysed at different time points of 0, 1week, 2weeks, 4weeks, 12 weeks and 24 weeks. In all time points, viability and cell recovery (Figures 9A and 9B) is shown to be >98%.

Further, stability of different doses of 25M (million), 50M (million) and 75M (million) products are analysed by freezing the IMP at 25M cells per ml in CryoStor 5 in CZ vials of 5ml and stored in-196°C for different time points upto 12 months.

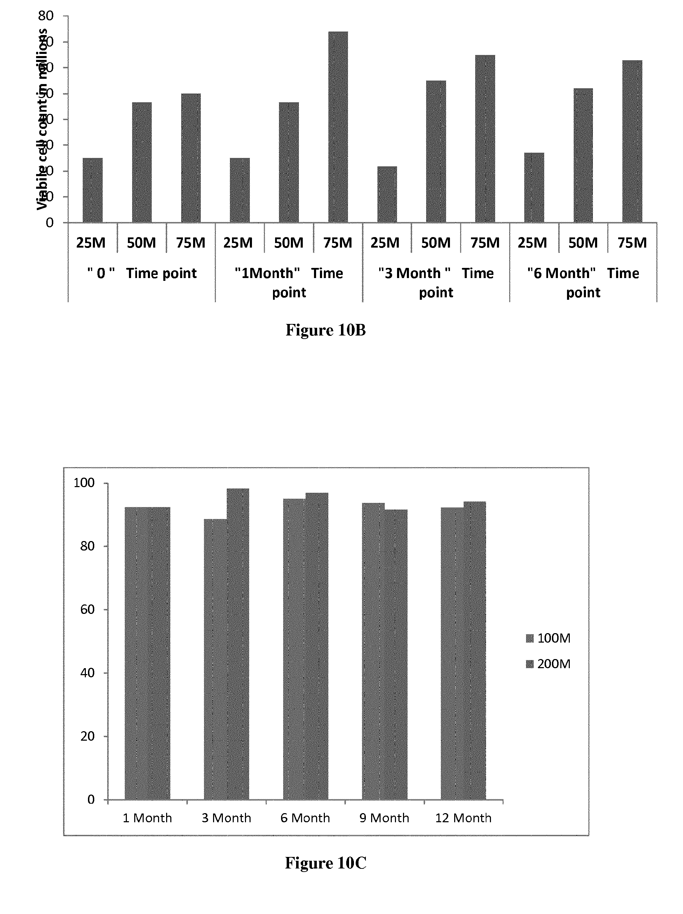

All the doses of 25M, 50M and 75M cells frozen at 25M cells per ml have shown >85% viability and cell recovery (Figures 10A and 10B) during all the time points upto 12 months. Figure 10C shows the viability by 7AAD for 25M cells per ml of CS5 freezing mixture of different dose of 100 and 200 million for 12 months stored at -196°C.

The cells so manufactured using the current process of the present disclosure are hypoimmunogeneic and immunomodulatory. These characteristics of the cells are further analyzed in the below examples.

### EXAMPLE 5:

### Hypoimmunogenic, immunomodulatory and angiogenic properties of the IMP:

The MSCs of the IMP developed by the current process exhibit hypoimmunogenic, immunomodulatory and angiogenic properties.

### Estimation of immunogenicity and immunosuppressive capacity of IMP:

Mesenchymal stromal cells (MSCs) have the potential to be used as off-the-shelf therapeutic agents across the major histocompatibility complex (MHC/HLA) barrier due to their important property of immunomodulation. They suppress proliferation of mitogen activated or allo-activated lymphocytes. MSCs are therefore inefficient at stimulating a T cell response in an allogeneic recipient, and are well tolerated when transplanted across HLA barriers in humans. Mechanism of immunosuppression by MSC is predominantly through secretion of immunoregulatory cytokines. Prostaglandin-E2 (PGE-2) is one of the important regulators of MSC-mediated immunosuppression.
The immunosuppressive capacity and the amount of PGE-2 produced by the final product was estimated to determine the immunological potency of the IMP.

### Immunosuppressive capacity of the IMP was determined by the following method:

The immunosuppressive properties of the IMP cells obtained from the process of the current disclosure are determined testing their ability to suppress a mixed lymphocyte reaction (MLR). MLRs are established using peripheral blood mononuclear cells (PBMC) from HLA-mismatched donors and cultured in either the presence or absence of growth-arrested MSC at different MSC:MLR ratios. The protocol for immunosuppression assays is as follows:
Mitomycin C-treated cells of the IMP were seeded in 96-well plates at varying cell numbers in the range 1.6x10⁵-2.5x10⁴ cells/well and allowed to attach overnight. A one-way MLR at a stimulator : responder ratio of 1:2.5 was then added to each well. To set up the MLR, 2x10⁵ responder PBMC were mixed with 8x10⁴ allogeneic stimulator PBMCs that had been previously treated with 25 mg/ml mitomycin C for 0.5 hours. The cultures were maintained for 5 days, during which they were pulsed with 5-bromo-2-deoxyuridine (BrdU) for the final 24 h. Cell proliferation was measured using a colorimetric immunoassay kit (Calbiochem) for the quantification of BrdU incorporation, according to the manufacturer's instructions. A set of MLR wells cultured in the absence of IMP cells was considered to be the proliferation control. For all assays, treatments were performed in triplicate in RPMI 1640 medium (Invitrogen) supplemented with 10% FBS (Hyclone), 2mM glutamine (Invitrogen) and 0.05 mM b-mercaptoethanol (Sigma-Aldrich).

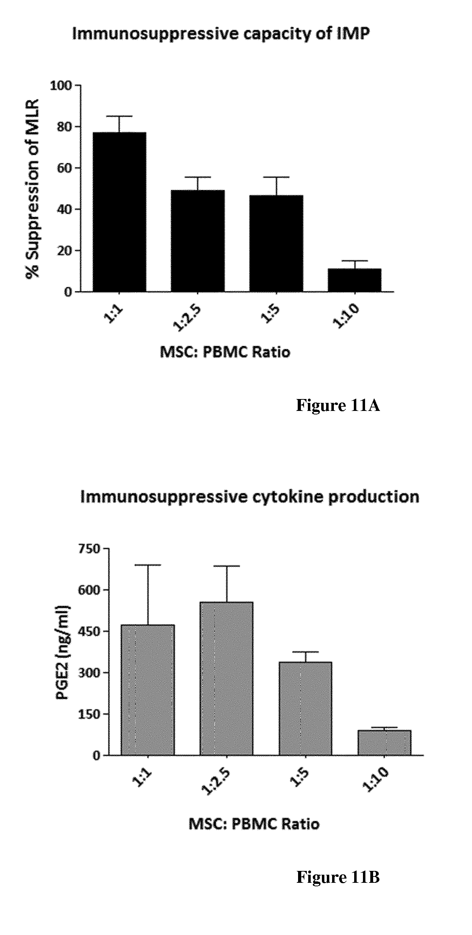

The results indicate that the IMP suppresses the proliferation of allogeneic lymphocytes in an MLR in a dose dependent manner (Figure 11A).

### Estimation of PGE-2:

MLRs were established using peripheral blood mononuclear cells (PBMC) from HLA-mismatched donors and cultured in either the presence or absence of growth-arrested MSC at different MSC : MLR ratios, using the protocol described above. At the end of 5 days of incubation, the supernatants from the IMP-MLR co-cultures were collected from the wells of the 96 well plates, collected media was spun down at 1500 rpm and stored at -80°C until use. Levels of PGE-2 in the supernatants were assayed using a competitive immunoassay kit (PGE-2 EIA kit, Enzo Lifesciences) for the quantitative determination of PGE-2, according to the manufacturer's instructions.

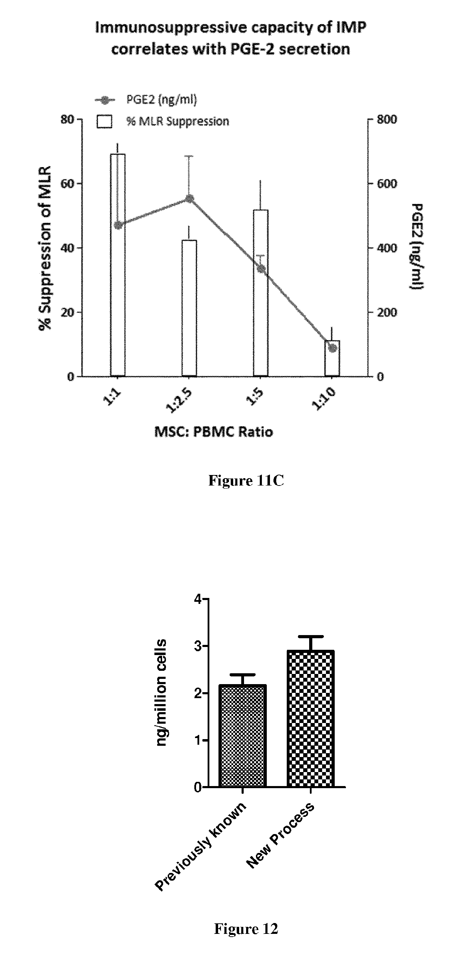

The results indicate that the IMP comprising cells of the current disclosure produces large quantities of PGE-2 when cultured in the presence of lymphocytes (Figure 11B). Secretion of PGE-2 shows a clear dose response, varying with the ratio of IMP cells to allogeneic PBMC. A positive correlation is seen between the amount of PGE-2 produced and the immunosuppressive capacity of the IMP, with greater immunosuppression obtained with higher amounts of PGE-2 (Figure 11C).

The IMP comprising cells of the current disclosure therefore has potent immunosuppressive capacity, and secreted large amounts of the immunoregulatory cytokine PGE-2. These properties make the IMP suitable for transplantation in an unrelated recipient, as well as for treatment of immune-related diseases such as graft versus host disease (GvHD) and other autoimmune diseases.
The amount of VEGF and sGAG produced by the final product is estimated for analyzing the potency of the IMP for Critical Limb Ischemia (CLI) and Osteoarthritis (OA) respectively.

### Estimation of VEGF:

The IMP obtained by following the process of the instant disclosure [having confluency based media change regime, washing process of Wash-II and taking into account the master and seed mix preparation of Example 1] is thawed at passage 5 and plated at a density of 1 million in a T75 flask. The conditioned medium is collected at 48 hours and 72 hours and the VEGF content is estimated by ELISA. This is compared with a previously known process of obtaining the final cell composition, wherein the process media changes are done based on no. of days as opposed to confluencies, washing process of Wash-I is followed and wherein no master and seed mix preparation are carried out.

The results indicating the difference in levels of VEGF is provided by Figure 12, when the previously known process and the process of the present disclosure are followed, wherein the VEGF content is enhanced in the cells obtained by following the process of the present disclosure.

Following is the protocol for the estimation of the VEGF for angiogenic potency:
VEGF being the potent angiogenic marker and their release by BM MSCs supported the idea that the paracrine mechanisms underpinned the biological effects of long-term angiogenesis in patients. Here we estimated the amount of VEGF in the conditioned medium of our IMP (BM MSCs) - P5 cultures. BM MSCs were plated at the density of 1X10⁶ cells in a T-75 flask (BD) in duplicates. Conditioned medium was collected at the end of 48 and 72 hours from the T 75 flasks which is fed with DMEM-KO, 10%FBS, glutamine (Glutamax) (100U/ml), Penstrep(50 to 100U/ml penicillin and 50 to 100µg/ml streptomycin) and 2ng/ml bFGF. Conditioned medium was also collected from the large scale production batches which were cultured in cell stacks on the day of harvest at 80-90% confluency at Passage 5. Collected media was spun down at 1500 rpm and filtered with a 0.22µ syringe filter (Millipore) stored at -80°C until use.
Human VEGF Quantikine ELISA Kit (R&D Systems, Minneapolis, MN) was used according to the directions of the manufacturer. 200 µl of conditioned medium was used for the assay. Separate standards were included for each run. The absorbance was read at 450nm using the Spectramax M3 plate reader.

### Estimation of sGAG:

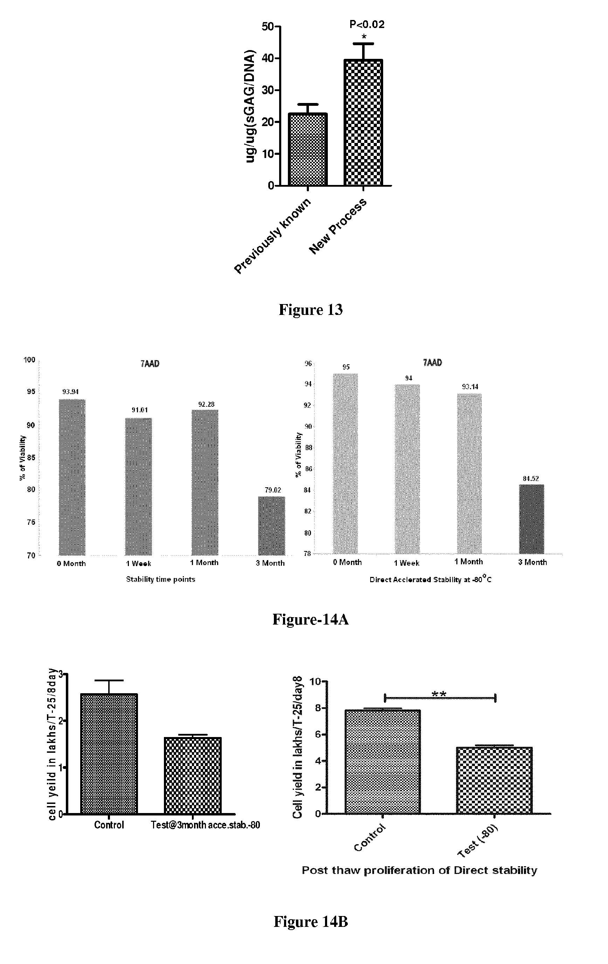

The IMP obtained by following the process of the instant disclosure [having confluency based media change regime, washing process of Wash-II and taking into account the master and seed mix preparation of Example 1] is thawed at passage 5 and induced for chondro differentiation. After 21 days of differentiation, sGAG and DNA is estimated. The final GAG value is normalized to the DNA content. This is compared with a previously known process of obtaining the final cell composition, wherein the process media changes are done based on no. of days as opposed to confluencies, washing process of Wash-I is followed and wherein no master and seed mix preparation are carried out (Figure 13). As can be noted, the value for the previously known process on an average was 22.54 ± 2.983 ug/ug of DNA, whereas for the process of the instant disclosure, the value on an average was 39.38 ± 5.212 ug/ug of DNA, thereby making the values significantly enhanced.

As chondrocytes are required for cartilage formation and regeneration, the potency of BM MSCs for chondrocyte differentiation was measured.. sGAG produced by the chondrocyte help stabilize the cartilage structure, and therefore it would be important to determine if the MSCs produced by the method of the instant disclosure can differentiate to chondrocyte and produce sGAG under in-vitro condition. The biochemical assay for measuring the sGAG is established, which is one of the important cartilage components and comprises of about 3 - 6% of total cartilage components. Following is the protocol for estimating GAG content:
1. Cells were plated at the density of 1000 cells/cm.sq in 6 well plates(1 set of control and 1 set for differentiation).
2. Assay was performed in duplicate comprising 2 wells for sGAG estimation,2 wells for DNA estimation,1 well for RNA and 1 well for staining.
3. At 70% confluency the cells were trypsinized from the control wells and the pellets were stored at -80 degrees along with the spent media from sGAG wells.
4. Chondrogenic differentiation was induced using STEM PRO kit from GIBCO. Media change was performed every 48 hours.
5. And the cells were allowed to differentiate upto 21 days.
   On day 21 the cells were trypsinized and collected spun down at 1100rpm and store the dry pellet at -80 degrees until assayed.
6. On the day of the assay, samples were thawed on ice and sGAG was estimated using the BLYSCAN Kit protocol. According to the protocol the cell pellet was lysed with papain at 65 degrees for 3 hours followed by the staining with dimethyl methylene blue. sGAG reacts with dye in acidic pH and give GAG - dye complex which displays a difference in absorption maxima at 550nm.

The results indicating the difference in levels of sGAG are provided by Figure 13, when the previously known process and the process of the present disclosure are followed, wherein the sGAG content is enhanced in the cells obtained by following the process of the present disclosure.

### EXAMPLE 6:

### Direct stability at -80°C:

Efficiency of the MSCs of the instant disclosure after cryopreservation is assessed by freeze-thaw viability, cell recovery, post-thaw proliferation, CFU-F and differentiation potential. The longer stability of cryopreserved MSCs depends on cryopreservation medium and process or method of cryopreservation.

Accelerated Stability: Standard practice of accelerated stability study involves control rate freezing (-80°C) and then shifting vials to liquid nitrogen storage and again after one week from liquid nitrogen storage to -80°C deep freezing to establish accelerated stability conditions. Usually, after control rate freezing, the MSCs must be placed in liquid nitrogen and the cells are highly unstable at -80°C after CRF.

Direct Stability: On the other hand, the instant disclosure provides for direct stability concept wherein higher freezing concentration of MSCs plus the process/conditions of stability enables a superior performance, efficiency and stability than standard accelerated method. Overall, the study enables to establish the shelf life period of active substances (MSCs) of drug product in CZ vials at direct accelerated conditions of -80°C after CRF without any exposure of liquid nitrogen of -196°C. These studies have been carried out when the MSCs were stored in CS5 media in CZ vials at high density of 25 million cells per ml.

The MSCs show promising or superior results than previously known accelerated stability studies for short term storage mostly for transportation of the product. In most of other cell therapy industries, control rate freezing ends at - 135°C, and thereafter the MSCs are moved to liquid nitrogen. On the contrary, stabilization of MSCs at -80°C is unique and provides advantages for short time storage and transportation without use of liquid nitrogen which is costly.

### 7AAD viability of accelerated procedure and direct stability procedure

Protocol: BM-MSCs were harvested and re-suspended in wash buffer at a concentration of 1x10⁶ cells/mL. Wash buffer consisted of phosphate buffer saline (PBS) supplemented with 1% (v/v) FBS and 1% (w/v) sodium azide. Cell viability was measured by flow cytometry using 7- amino actinomycin D (7AAD, which can penetrate through cell membrane of dead cells). The 100 µl of cell suspension were incubated in the dark for 10 minutes at room temperature with saturating concentrations of 7AAD of 3 µl in one well of 96 well plate and unstained cells in another well as auto. Run the flow cytometry first with auto to set and adjust the voltage and compensation parameters to fall within the histogram. Run the test samples as 7AAD while fixing those previous setting.

Result: The results (Figure 14A) show that 3rd month time point of direct stability has 84.52% of viability, whereas accelerated conditions has 79.02%.

### Post-thaw proliferation at 3^{rd} month time point

Accelerated stability culture conditions: Post-thaw proliferation study done in T-25 flask (n=3)p=0.0991, 1K seeding density, Harvest culture age:8^{th} day. Direct stability culture conditions: post-thaw proliferation study done in T-25 flask (n=3),** p<0.001, 1K seeding. Control represents post-thaw proliferation of 1 million cell vials of BM-MSCs (cryo-vial) of same batch and cryopreserved in regular standard procedure,. Overall results show that post-thaw proliferation rate and yield were higher in direct stability compared to accelerated conditions, harvest culture age: 8^{th} day.

Results: Post thaw proliferation (Figure 14B)_of direct stability study has shown better viability than accelerated stability.

### CFU-F Assay of accelerated procedure and direct stability procedure

Protocol: For CFU-F assay, 100 MSCs were plated in from direct stability study vials and accelerated stability vials (n=2 of each condition) on a 100 mm² cell culture dish. The plates were stained with crystal violet after 14 days of incubation and visible colonies were counted.

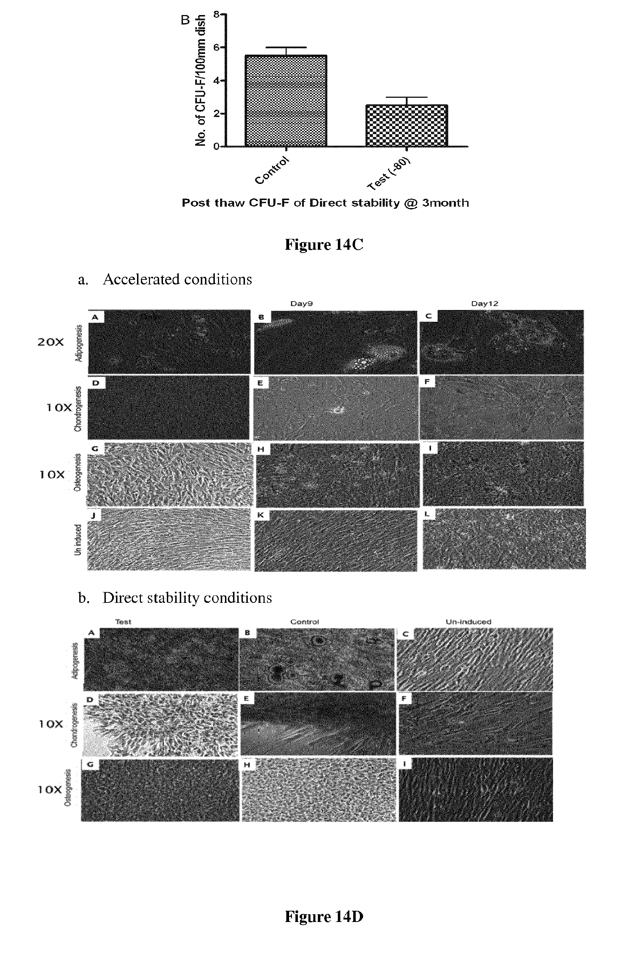

Results: CFU-F of accelerated conditions unable to show any colonies even at 21 day time point (Figure 14C).

### Tri-lineage potential studies

Protocol: Osteoblast differentiation of BM-MSCs was induced by supplementing the cells with 10-⁸M dexamethasone (Sigma-Aldrich), 30µ g/mL ascorbic acid (Sigma-Aldrich) and 10 mM β-glycerophosphate (Sigma-Aldrich) for 3 weeks. Calcium accumulation was assessed by Von Kossa Staining. The differentiated cells were washed with PBS and fixed with 10% formalin for 30 minutes. The fixed cells were incubated with 5% AgNO₃ for 60 minutes under UV light and then treated with 2.5% sodium thiosulphate for 5 minutes and images were captured.

Protocol: To induce adipogenic differentiation, at BM-MSCs were supplemented with 1 µM dexamethasone, 0.5 mM isobutylmethylxanthine, 1 µg/mL insulin and 100 µM indomethacin (all Sigma-Aldrich) for 21 days. Then supernatant were aspirated and cells were fixed in 10% formalin for 20 minutes. Further 200 µl of oil red O staining solution was added and incubated for 10 minutes at room temperature. The cells were rinsed five times with distilled water and images were captured.

Protocol: To induce chondrogenic differentiation, at the BM-MSCs of direct stability and accelerated stability vials were cultured in STEMPRO (Invitrogen) chondrogenesis differentiation medium (Chondrocyte differentiation basal medium with chondrogenesis supplement). Chondrogenesis differentiation medium was replenished every 3 days. For staining, cells were fixed with 4% formalin for 30 minutes. Safranine O staining solution was added and incubated for 5 minutes. The cells were rinsed with distilled water and images were captured. BM-MSCs differentiated towards osteogenic, chondrogenic and adipogenic images were captured using Nikon Eclipse 90i microscope (Nikon Corporation, Japan, www.nikon.com) and Image-Pro Express software (Media Cybernetics, Inc, Silver Spring, MD, www.mediacy.com).

Summary: It is observed that the direct stability studies shows higher viability compared to accelerated stability in terms of 7AAD, CFU-F and multilineage differentiation ability than accelerated conditions.

Results: Post thaw proliferation (Figure 14D) of direct stability study has shown to be better than accelerated stability.

## Claims

1. A process for culturing of mesenchymal stromal cells, said process comprising act of:
a. allowing the mesenchymal stromal cells cultured till a first predetermined passage, to expand to a second predetermined passage in presence of culture media comprising basic fibroblast growth factor (bFGF);
wherein said expansion is carried out by contacting the cells with said culture media when said cells achieve at least one pre-determined confluency; and wherein the process increases number of the cells at the end of the second predetermined passage by 2000 folds when compared to number of the cells at the first predetermined passage.

2. The process as claimed in claim 1, wherein the increase in the number of cells occurs in a maximum period of 21 days, and wherein the at least one pre-determined confluency is selected from ranges of confluencies comprising about 30% to about 40%; about 40% to about 50%; and about 60% to about 70%, or any combination thereof.

3. The process as claimed in claim 1, wherein the cells at the first predetermined passage are allowed to expand to the second predetermined passage by consecutive passages; and wherein the first predetermined passage ranges from passage 2 to passage 9; and the second predetermined passage ranges from passage 5 to passage 10, preferably wherein the first predetermined passage is passage 3; and the second predetermined passage is passage 5.

4. The process as claimed in claim 1, wherein number of the cells seeded for said expansion ranges from about 0.6 million to about 0.7 million cells; and number of the cells obtained after said expansion is at least about 1800 million cells; and wherein the process increases the number of cells by at least 2000 folds.

5. The process as claimed in claim 1, wherein components of the culture media are Dulbecco's Modified Eagle Medium-KnockOut [DMEM-KO] at a concentration ranging from about 75% to 95%, Fetal Bovine Serum [FBS] at a concentration ranging from about 5% to 15%, Glutamine at a concentration ranging from about 0.5% to 2%, Pen-Strep having penicillin at a concentration of about 50 to about 100 U/ml and streptomycin at a concentration of about 50 to about 100 µg/ml, and basic Fibroblast Growth Factor (bFGF) at a concentration ranging from about 0.5 ng/ml to 5 ng/ml.

6. The process as claimed in claim 5, wherein the culture media is prepared by a process of master mixing of the components in a manner so as to avoid volumetric error and provide uniform distribution of the bFGF within multiple aliquots of the media, said process comprising acts of:
a. preparing 'X' Ltrs of the culture media comprising the DMEM-KO, FBS, Glutamine and Pen-Strep, devoid of bFGF, and dispensing into 'X' 1Ltr containers and labelling the containers from 1 to 'X';
b. withdrawing 'Z' ml of media from container No. 1, and dispensing into a new sterile container labelled No. 'X+1';
c. adding a predetermined quantity of bFGF to the '1Ltr minus(-) Z' ml of media of container No. 1 and mixing well to obtain bFGF master mix;
d. separately withdrawing an equal quantity of media from each of the containers labelled 2 to 'X' and adding to the 'Z' ml of media in container No. 'X+1', thereby making the total media volume of container No. 'X+1' as 'B' ml; and
e. separately withdrawing a predetermined quantity of bFGF master mix obtained in step (c) and adding to each 'B' ml media in containers 2 to 'X' thereby making the volume in each of the said containers equal and preparing multiple aliquots of the culture media.

7. The process as claimed in claim 6, wherein the predetermined quantity of bFGF in step (c) ranges from about 1 ng/ml to about 3 ng/ml of the media, preferably about 2 ng/ml of the media.

8. The process as claimed in claim 6, wherein in step (d), the equal quantity of the media is withdrawn in a manner such that upon completion of said step (d), each of the containers 2 to 'X' and 'X+1' contain equal amounts of media, and/or wherein in step (e), the predetermined quantity of the bFGF is added in a manner such that upon completion of said step (e), each of the containers 2 to 'X' and 'X+1' contain 1L of media.

9. The process as claimed in claim 1, wherein the expansion by contacting the cells with the culture media comprises acts of:
a. expanding the cultured cells into a consecutive passage by seeding the cells at a seeding density ranging from about 1000 cell/cm² to about 7000 cells/cm² and providing the culture media to the seeded cells; and
b. allowing the cells to reach a predetermined confluency of about 40% to about 50%, and replacing the culture media to obtain the expanded cells;
wherein, the culture media employed throughout the expansion is at a concentration ranging from about 0.15 to about 0.35 ml/ cm² or
wherein the expansion by contacting the cells with the culture media comprises acts of:
a. expanding the cultured cells into a consecutive passage by seeding the cells at a seeding density ranging from about 1000 cell/cm² to about 7000 cells/cm² and providing the culture media to the seeded cells;
b. allowing the cells to reach a predetermined confluency of about 30% to about 40%, and adding the culture media to the cells without removing the spent media; and
c. allowing the cells to reach a second predetermined confluency of about 60% to about 70%, and replacing the culture media to obtain the expanded cells;
wherein, the culture media employed throughout the expansion is at a concentration ranging from about 0.15 to about 0.35 ml/ cm² or
wherein the expansion by contacting the cells with the culture media comprises acts of:
a. expanding the cultured cells into a consecutive passage by seeding the cells at a seeding density ranging from about 1000 cell/cm² to about 7000 cells/cm² and providing the culture media to the seeded cells;
b. allowing the cells to reach a predetermined confluency of about 40% to about 50%, and replacing the culture media of the cells;
c. further expanding the cells of step (b) into a second consecutive passage by seeding the cells at a seeding density ranging from about ranging from about 1000 cell/cm to about 7000 cells/cm and providing the culture media to the seeded cells;
d. allowing the cells to reach a predetermined confluency of about 30% to about 40%, and adding the culture media to the cells without removing the spent media; and
e. allowing the cells to reach a second predetermined confluency of about 60% to about 70%, and replacing the culture media to obtain the expanded cells;
wherein, the culture media employed throughout the expansion is at a concentration ranging from about 0.15 to about 0.35 ml/ cm² or
wherein the expansion by contacting the cells with the culture media comprises acts of:
a. expanding the cells cultured to passage 3 to expand into passage 4 by seeding the passage 3 cells at a seeding density ranging from about 1000 cell/cm² to about 7000 cells/cm² and providing the culture media to the seeded cells;
b. allowing the cells to reach a predetermined confluency of about 40% to about 50%, and replacing the culture media;
c. further expanding the cells obtained at the end of step (b) into passage 5 by seeding the cells at a seeding density ranging from about 1000 cell/cm² to about 7000 cells/cm² and providing the culture media to the seeded cells;
d. allowing the cells to reach a predetermined confluency of about 30% to about 40%, and adding the culture media without removing the spent media; and
e. allowing the cells to reach a second predetermined confluency of about 60% to about 70%, and replacing the culture media to obtain the expanded cells;
wherein, the culture media employed throughout the expansion is at a concentration ranging from about 0.15 to about 0.35 ml/ cm².

10. The process as claimed in claim 9 comprising acts a. and b., wherein the expansion increases the number of cells by at least 30 folds, or
the process as claimed in claim 9 comprising acts of a., b., and c, wherein the expansion increases the number of cells by at least 40 folds, or
the process as claimed in claim 9 comprising acts of a. to e., wherein act a. comprises expanding the cultured cells into a consecutive passage by seeding the cells at a seeding density ranging from about 1000 cell/cm² to about 7000 cells/cm² and providing the culture media to the seeded cells and act e comprises allowing the cells to reach a second predetermined confluency of about 60 % to about 70 %, and replacing the culture media to obtain the expanded cells, wherein the expansion increases the number of cells by at least 2000 folds.

11. The process as claimed in claim 1, wherein the cells obtained after said culturing are subjected to freezing with a freezing mixture at cell density ranging from about 5 million cells to about 25 million cells per ml of the freezing mixture, preferably wherein the freezing is carried out at a temperature ranging from about -75 degrees Celsius to about -85 degrees Celsius or at a temperature ranging from about -190 degrees Celsius to about -200 degrees Celsius; and wherein the freezing mixture comprises a cryopreservant at a concentration ranging from about 2% to about 15%, preferably about 5%; and wherein the cryopreservant is preferably DMSO.

12. The process as claimed in claim 11, wherein the freezing at said cell density allows retention of viability and post-thaw functionality.

13. The process as claimed in claim 1, wherein said process comprises act of:
a. allowing the mesenchymal stromal cells cultured till passage 3, to expand to passage 5 in presence of culture media comprising basic fibroblast growth factor (bFGF);
wherein said expansion is carried out by contacting the cells with said culture media when said cells achieve at least one pre-determined confluency; and wherein the process increases number of the cells at the end of passage 5 by at least 2000 folds when compared to number of the cells at the passage 3, preferably wherein the at least one pre-determined confluency is selected from ranges of confluencies comprising about 30% to about 40%; about 40% to about 50%; and about 60% to about 70%, or any combination thereof.

14. The process as claimed in claim 1, wherein the cells obtained upon said culturing process are further subjected to a combination of centrifugation and wash process prior to being formulated into a predetermined dosage form for clinical or therapeutic application; and wherein the said combination reduces the levels of bovine serum albumin [BSA] present to an amount of below 50ng/ml for said predetermined dosage, wherein said BSA is provided to the cells during the culturing process by the FBS in the culture media.

15. A method of reducing amount of BSA to a level of below 50ng/ml for a dosage formulated for clinical or therapeutic application, said dosage comprising mesenchymal stromal cells obtained by culturing of said cells, said method comprising act of subjecting the composition to a combination of centrifugation and washing with phosphate buffer saline to reduce the amount of said BSA.

16. The method as claimed in claim 15 preferably wherein the BSA at an amount of above 50ng/ml is provided to the cells during the culturing of said cells involving use of components of bovine origin; and wherein the level of below 50ng/ml is regardless of the number of the cells in the dosage.

17. The method as claimed in claim 15, wherein the method comprises acts of:
a. subjecting the cells to a first centrifugation at speed ranging from about 1200 rpm to about 1800 rpm for time period ranging from about 7 minutes to about 10 minutes;
b. subjecting the centrifuged cells to a first washing with about 20 ml DPBS for cell numbers in the range of about 0.6million to about 6 million, and re-centrifuging the washed cells at speed ranging from about 1200 rpm to about 1800 rpm for time period of about 10 minutes;
c. subjecting the re-centrifuged cells to a second washing with about 7 to about 9 ml of DPBS for cell numbers in the range of about 600 to about 6 million, followed by optional pooling of the cell samples; and
d. re-centrifuging the washed cells from step (c) at speed ranging from about 1200 rpm to about 1800 rpm for time period ranging from about 5 minutes to about 7 minutes to obtain the cells having BSA at a level of below 50ng/ml.

18. A process for storing mesenchymal stromal cells, said process comprising step of subjecting the cells to a freezing mixture at a cell density ranging from about 5 million cells to about 25 million cells per ml of the freezing mixture, and storing the cells at a temperature ranging from about -75 degrees Celsius to about -85 degrees Celsius or at a temperature ranging from about -190 degrees Celsius to about -200 degrees Celsius.

19. The process as claimed in claim 18, wherein the storing of mesenchymal stromal cells in a freezing mixture at a cell density ranging from about 5 million cells to about 25 million cells per ml of the freezing mixture, and at a temperature ranging from about -75 degrees Celsius to about -85 degrees Celsius, allows retention of viability and post-thaw functionality for a period of at least 1 week without liquid nitrogen, or wherein the storing of mesenchymal stromal cells in a freezing mixture at a cell density ranging from about 5 million cells to about 25 million cells per ml of the freezing mixture, and at a temperature ranging from about -190 degrees Celsius to about -200 degrees Celsius, allows retention of viability and post-thaw functionality for a period of at least 1 month.

20. The process as claimed in claim 18, wherein the freezing mixture comprises a cryopreservant at a concentration ranging from about 4% to about 11%, preferably about 5%; and wherein the cryopreservant is preferably DMSO.

21. The process as claimed in claim 18, wherein the freezing at said cell density allows retention of viability and post-thaw functionality, and wherein the process eliminates reconstitution of the cells prior to administration for a therapeutic purpose to a subject in need thereof.

22. The process as claimed in claim 18, wherein the mesenchymal stromal cells are obtained by culturing of said cells by process as claimed in claim 1.
